# EUROPEAN PATENT APPLICATION

(11) **EP 1 779 867 A1**
(43) Date of publication of application: **02.05.2007**
(21) Application number: 05757851.0
(22) Date of filing: 01.07.2005
(51) Int. Cl.: A61K 45/00, A61K 31/27, A61K 31/445, A61K 31/4525, A61P 25/00, A61P 25/28, A61P 43/00, G01N 33/15, G01N 33/50

(54) **NERVE REVENERATION PROMOTER**

(30) Priority: 01.07.2004 JP 2004195993
(71) Applicant: Eisai R&D Management Co., Ltd., Tokyo 112-8088 (JP)
(72) Inventor: TERAMOTO, Tetsuyuki, EISAI CO.,LTD.Tsukuba R.Lab., Tsukuba-shi, Iberaki 300-2635 (JP); YAMAUCHI, Toshihiko, EISAI CO.,LTD.Tsukuba R.Lab., Tsukuba-shi, Iberaki 300-2635 (JP); KOTANI, Sadaharu, EISAI CO.,LTD.Tsukuba R.Lab., Tsukuba-shi, Iberaki 300-2635 (JP)
(74) Representative: Woods, Geoffrey Corlett
(86) International application number: PCT/JP2005/012636
(87) International publication number: WO 2006/004201

(57) **Abstract**

A nerve regeneration stimulator comprising a compound having a cholinesterase inhibitory activity, a pharmacologically acceptable salt thereof or a solvate thereof.

## Description

### FIELD OF THE INVENTION

The present invention relates to a nerve regeneration stimulator comprising a compound having a cholinesterase inhibitory activity and to method for screening the nerve regeneration stimulator.

### BACKGROUND ART

Cholinesterase is distributed over the body and is a generic name for enzymes that hydrolyze cholinesters such as acetylcholine that acts as a nerve transmitter. Such enzymatic activity is inhibited with physostigmine (10⁻⁵ mol/L). Two types of cholinesterases are known, namely, true cholinesterase that specifically hydrolyzes acetylcholine that exists in nerve tissues, red blood cells, muscles and elsewhere and pseudocholinesterase that hydrolyzes cholinesters as well as other various esters that exist in serum, liver, pancreas and elsewhere. The former is called acetylcholinesterase (EC3.1.1.7) that hydrolyzes acetylcholine faster than other cholinesters and that is susceptible to substrate inhibition. On the other hand, the latter has the property of hydrolyzing various cholinesters other than acetylcholine as well as esters that do not contain choline.

Cholinesterase inhibitors *suppress in vivo* acetylcholine hydrolysis by cholinesterase, increase acetylcholine in synaptic cleft and enhance acetylcholine transmission. In particular, donepezil hydrochloride that acts as acetylcholinesterase inhibitor increases acetylcholine in the brain and is used extensively as a drug for treating Alzheimer's senile dementia (Japanese Patent No. 2578475).

Hippocampus is a site in the brain that plays an important role in formation and maintenance of memory (Eric R. Kandel, The Molecular Biology of Memory Storage: A Dialogue Between Genes and Synapses, Science, 294:1030-1038, 2001). Recently, neural stem cells (that develop into nerve cells) were found to exist in the dentate gyrus (an area of the neural circuit in the adult hippocampus) and their neurogenesis was found to be maintained all life through ( Fred H. Gage, Mammalian Neural Stem Cells, Science, 287: 1433-1438, 2000, Arturo Alvarez-Buylla, Bettina Seri and Fiona Doetsch, Identification of neural stem cells in the adult vertebrate brain, Brain Research Bulletin, 57:751-758, 2002). Target molecules that regulate the nerve regeneration, however, have not been fully understood.

Acetylcholine nerve system projects to the dentate gyrus and plays an important role in formation and maintenance of memory (Michelle L. Gilmor, Scott E. Counts, Ronald G. Wiley, and Allan I. Levey, Coordinate Expression of the Vesicular Acetylcholine Transporter and Choline Acetyltransferase Following Septohippocampal Pathway Lesions, J Neurochem. 71:2411-2420, 1998). It has been reported in The Society for Neuroscience that nerve regeneration in the dentate gyrus is decreased when this acetylcholine nerve system is destructed (C.M. Cooper-Kuhn, J. Winkler, H. Kuhn, ALTERED NEUROGENESIS AFTER CHOLINERGIC FOREBRAIN LESION IN THE ADULT RAT. Soc. Neurosci. Abstr., Program No. 348. 9, 2003). A drug that activates acetylcholine nerve system projecting to the dentate gyrus and that stimulates nerve regeneration in the dentate gyrus (nerve regeneration stimulator) has not yet been reported.

"Social anxiety disorder" is a condition that causes strong nervousness, anxiety, fear or the like, for example, in front of people, that results in interference with social life. Recent studies say that the chance of people to suffer from social anxiety disorder is approximately 10%, with little difference between Japan and the Western countries. Social anxiety disorder is one of the most popular mental illnesses along with depression.

Social anxiety disorders can be classified into generalized anxiety, panic disorder, post-traumatic stress disorder (PTSD), social phobia, specific phobia, obsessive-compulsive disorder and the like. Although symptoms of social anxiety disorders differ depending on each disorder, common symptoms include: impractically excessive anxiety that lasts for a long period of time, restlessness, hypersensitiveness, sleep disorder, excessive cautiousness, autonomic nervousness, repulsion, fear and repetitive obsessive idea or behavior.

Traditionally, drug treatments for social anxiety disorders have employed alcohol, barbituric acid, opiate, β-blocker and benzodiazepine minor tranquilizer, among which benzodiazepine has been used extensively for having the highest specificity and for being effective for various anxiety disorders. This drug is known to enhance the activity of γ-aminobutyric acid (GABA) that acts as an inhibitory transmitter in the brain. In addition, selective serotonin reuptake inhibitors (SSRIs) have been used for treating anxiety disorders since 1980s. SSRIs are widely used today because it does not show habit forming like benzodiazepine.

Although causes of social anxiety disorders are yet unknown, a combination of genetic predispositions and environmental factors is suggested to be involved in the onset thereof. Recent animal tests or clinical studies indicate a possibility that a decrease in nerve regeneration in the hippocampus by stress or trauma may be one of the causes. For example, patients suffering from posttraumatic stress disorder are reported to have a reduced volume of hippocampus compared to a healthy person (J. Douglas Bremner, et al., MRI-BASED MEASUREMENT OF HIPPOCAMPAL VOLUME IN POSTTRAUMATIC STRESS DISORDER. Am. J. Psychiatry 152: 973-981, 1995), and a decrease in the nerve regenerating reaction described above is suggested as one of the causes (J. Douglas Bremner, NEUROIMAGING STUDIES IN POST-TRAUMATIC STRESS DISORDER. Curr. Psychiatry Rep. 4: 254-263, 2002).

Administration of paroxetine as one of SSRIs for treating social anxiety disorders has been reported to increase the volume of hippocampus of PTSD patients (J. Douglas Bremner, NEUROIMAGING STUDIES IN POST-TRAUMATIC STRESS DISORDER. Curr. Psychiatry Rep. 4: 254-263, 2002). Furthermore, results from animal tests indicate that SSRIs stimulate nerve regeneration in the hippocampus (Jessica E. Malberg, et al., CHRONIC ANTIDEPRESSANT TREATMENT INCREASES NEUROGENESIS IN ADULT RAT HIPPOCAMPUS. J. Neurosci. 20: 9104-9110, 2000). Therefore, SSRIs may possibly exhibit treatment effects by stimulating nerve regeneration in hippocampus of PTSD patients.

### SUMMARY OF THE INVENTION

The present invention has an objective of providing a drug for stimulating nerve regeneration.

Based on the findings so far, the present inventor has proposed a hypothesis that a cholinesterase inhibitor that activates acetylcholine nerve system projecting to the dentate gyrus stimulates nerve regeneration in the dentate gyrus. Thus, this hypothesis was tested as follows. First, in order to objectively and simply assess the effect of stimulating nerve regeneration, and in order to efficiently find a nerve regeneration stimulator, a novel screening method using a non-human mammal comprising neural stem cells was established. The screening method of the invention uses 5-bromo-2'-deoxyuridine (BrdU) as a labeling marker for neural stem cells to enable immunohistochemical detection of nerve regeneration and thus is suitable for correctly screening a nerve regeneration stimulator.

The present inventor has used the screening method of the invention for keen examination, as a result of which a cholinesterase inhibitor was found to be effective in maintaining cells regenerating from neural stem cells present in the dentate gyrus, thereby completing the present invention. Thus, the present invention provides the followings.
(1) A nerve regeneration stimulator comprising a compound having a cholinesterase inhibitory activity, a pharmacologically acceptable salt thereof or a solvate thereof.
   An example of a compound having a cholinesterase inhibitory activity as described above includes a cyclic amine derivatives represented by the following general formula: 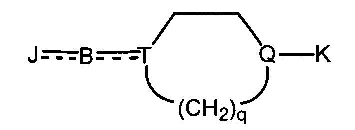 (wherein, J is:
   (a) a substituted or unsubstituted (1) phenyl group, (2) pyridyl group, (3) pyradyl group, (4) quinolyl group, (5) cyclohexyl group, (6) quinoxalyl group or (7) furyl group;
   (b) a monovalent or divalent group derived from a group selected from the group consisting of (1) indanyl, (2) indanonyl, (3) indenyl, (4) indenonyl, (5) indandionyl, (6) tetralonyl, (7) benzsuberonyl, (8) indanolyl, or (9) a group represented by formula 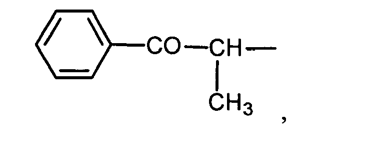 in all of which a phenyl group may be substituted;
   (c) a monovalent group derived from a cyclic amide compound;
   (d) a lower alkyl group; or
   (e) a group represented by formula R¹-CH = CH- (wherein R¹ is a hydrogen atom or a lower alkoxycarbonyl group),
      B is a group represented by formula 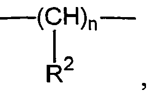 a group represented by formula 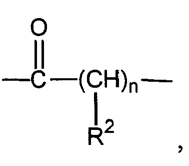 a group represented by formula 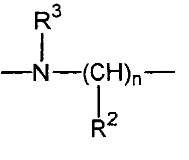 (wherein, R³ is a hydrogen atom, a lower alkyl group, an acyl group, a lower alkylsulfonyl group, a substituted or unsubstituted phenyl group or benzyl group), a group represented by formula 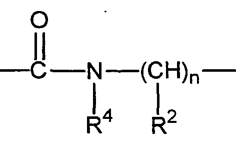 (wherein, R⁴ is a hydrogen atom, a lower alkyl group or a phenyl group), a group represented by formula 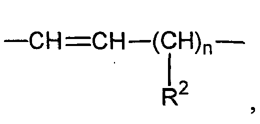 a group represented by formula 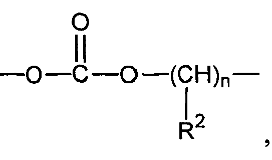 a group represented by formula 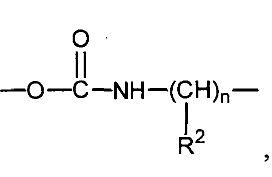 a group represented by formula 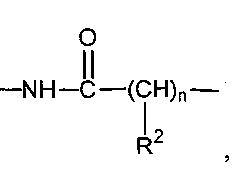 a group represented by formula 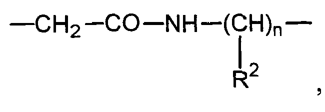 a group represented by formula 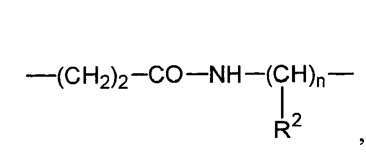 a group represented by formula 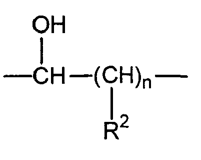 (wherein, n is 0 or an integer of 1 to 10, and R² is a hydrogen atom or a methyl group), a group represented by formula =(CH-CH=CH)_{b}- (wherein, b is an integer of 1 to 3), a group represented by formula =CH-(CH₂)_{c}- (wherein, c is 0 or an integer of 1 to 9), a group represented by formula =(CH-CH)_{d}= (wherein, d is 0 or an integer of 1 to 5), a group represented by formula 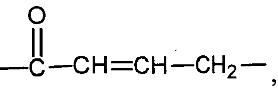 a group represented by formula 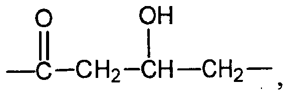 a group represented by formula 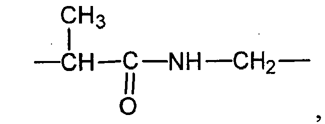 a group represented by formula 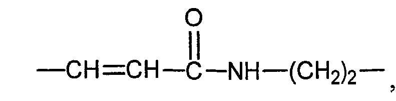 a group represented by formula -NH-, a group represented by formula -O-, a group represented by formula -S-, a dialkylaminoalkylcarbonyl group or a lower alkoxycarbonyl group,
      T is a nitrogen atom or a carbon atom,
      Q is a nitrogen atom, a carbon atom or a group represented by formula 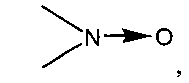 K is a hydrogen atom, a substituted or unsubstituted phenyl group, an arylalkyl group in which a phenyl group may be substituted, a cinnamyl group in which a phenyl group may be substituted, a lower alkyl group, a pyridylmethyl group, a cycloalkylalkyl group, an adamantanemethyl group, a furylmethyl group, a substituted or unsubstituted cycloalkyl group, a lower alkoxycarbonyl group or an acyl group,
      q is an integer of 1 to 3, and
      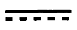 indicates a single bond or a double bond).

   Specifically, said J may be a group selected from the group consisting of substituted or unsubstituted (1) phenyl group, (2) pyridyl group, (3) pyradyl group, (4) quinolyl group, (5) cyclohexyl group, (6) quinoxalyl group and (7) furyl group. Furthermore, said J may be a monovalent group derived from a cyclic amide compound.
   The compound having a cholinesterase inhibitory activity in the nerve regeneration stimulator of the invention may be, for example, a cyclic amine derivative represented by the following general formula: 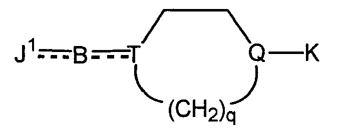 (wherein, J¹ is a monovalent or divalent group derived from a group selected from the group consisting of(1) indanyl, (2) indanonyl, (3) indenyl, (4) indenonyl, (5) indanedionyl, (6) tetralonyl, (7) benzosuberonyl, (8) indanolyl and (9) a group represented by formula 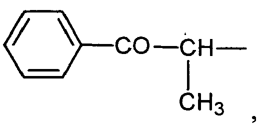 in all of which a phenyl group may be substituted, B is a group represented by formula 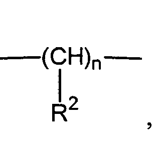 a group represented by formula 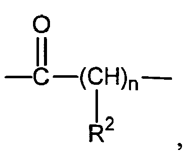 a group represented by formula 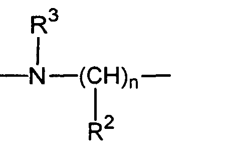 (wherein, R³ is a hydrogen atom, a lower alkyl group, an acyl group, a lower alkylsulfonyl group, a substituted or unsubstituted phenyl group or a benzyl group), a group represented by formula 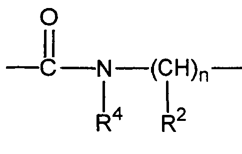 (wherein, R⁴ is a hydrogen atom, a lower alkyl group or a phenyl group), a group represented by formula 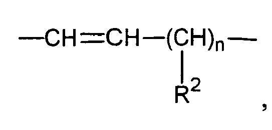 a group represented by formula 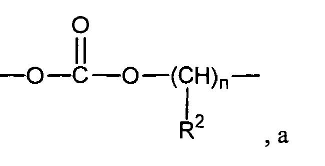 group represented by formula 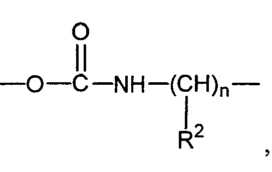 a group represented by formula 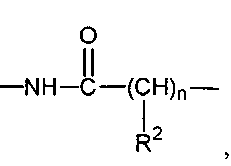 a group represented by formula 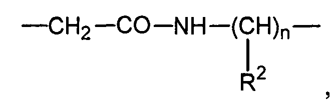 a group represented by formula 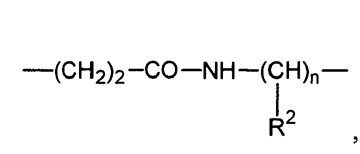 a group represented by formula 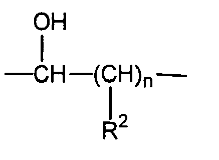 (wherein, n is 0 or an integer of 1 to 10, and R² is a hydrogen atom or a methyl group), a group represented by formula =(CH-CH=CH)_{b}- (wherein, b is an integer of 1 to 3), a group represented by formula =CH-(CH₂)_{c}- (wherein, c is 0 or an integer of 1 to 9), a group represented by formula =(CH-CH)_{d}= (wherein, d is 0 or an integer of 1 to 5), a group represented by formula 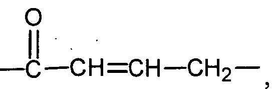 a group represented by formula 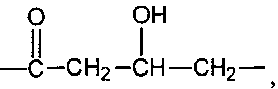 a group represented by formula 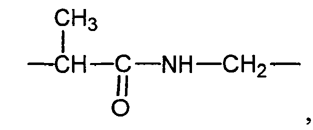 a group represented by formula 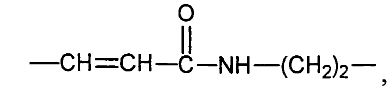 a group represented by formula -NH-, a group represented by formula -O-, a group represented by formula -S-, a dialkylaminoalkylcarbonyl group or a lower alkoxycarbonyl group,
   T is a nitrogen atom or a carbon atom,
   Q is a nitrogen atom, a carbon atom or a group represented by formula 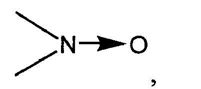 K is a hydrogen atom, a substituted or unsubstituted phenyl group, an arylalkyl group in which a phenyl group may be substituted, a cinnamyl group in which a phenyl group may be substituted, a lower alkyl group, a pyridylmethyl group, a cycloalkylalkyl group, an adamantanemethyl group, a furylmethyl group, a substituted or unsubstituted cycloalkyl group, a lower alkoxycarbonyl group or an acyl group,
   q is an integer of 1 to 3, and
   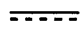 indicates a single bond or a double bond).
   Specifically, B may be a group represented by formula 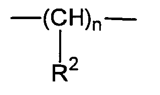 (wherein, n is 0 or an integer of 1 to 10 and R² is a hydrogen atom or a methyl group), a group represented by formula -CH=CH-(CH)ₙR²- (wherein, n is 0 or an integer of 1 to 10 and R² is a hydrogen atom or a methyl group), a group represented by formula =(CH-CH=CH)_{b}- (wherein, b is an integer of 1 to 3), a group represented by formula =CH-(CH₂)_{c}- (wherein, c is 0 or an integer of 1 to 9) or a group represented by formula =(CH-CH)_{d}= (wherein, d is 0 or an integer of 1 to 5).
   Furthermore, the compound having the cholinesterase inhibitory activity in the nerve regeneration stimulator of the invention may be, for example, a cyclic amine derivative represented by the following general formula: 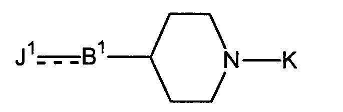 (wherein, J¹ is a monovalent or divalent group derived from a group selected from the group consisting of(1) indanyl, (2) indanonyl, (3) indenyl, (4) indenonyl, (5) indandionyl, (6) tetralonyl, (7) benzsuberonyl, (8) indanolyl and (9) a group represented by formula 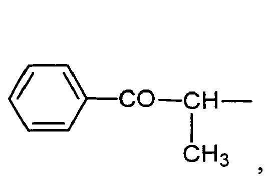 in all of which a phenyl group may be substituted,
   B¹ is a group represented by formula 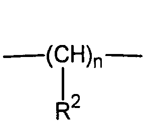 (wherein, n is 0 or an integer of 1 to 10, and R² is a hydrogen atom or a methyl group), a group represented by formula -CH=CH-(CH)ₙR²- (wherein, n is 0 or an integer of 1 to 10 and R² is a hydrogen atom or a methyl group), a group represented by formula =(CH-CH=CH)_{b}-(wherein, b is an integer of 1 to 3), a group represented by formula =CH-(CH₂)_{c}-(wherein, c is 0 or an integer of 1 to 9) or a group represented by formula =(CH-CH)_{d}= (wherein, d is 0 or an integer of 1 to 5), and
   K is a hydrogen atom, a substituted or unsubstituted phenyl group, an arylalkyl group in which a phenyl group may be substituted, a cinnamyl group in which a phenyl group may be substituted, a lower alkyl group, a pyridylmethyl group, a cycloalkylalkyl group, an adamantanemethyl group, a furylmethyl group, a substituted or unsubstituted cycloalkyl group, a lower alkoxycarbonyl group or an acyl group).
   Said K may be a substituted or unsubstituted arylalkyl group or phenyl group.
   Said J¹ may be a group selected from the group consisting of monovalent groups and divalent groups derived from indanonyl, indenyl and indandionyl. Furthermore, J¹ may be an indanonyl group that may have as a substituent a lower alkyl group with a carbon number 1 to 6 or a lower alkoxy group with a carbon number 1 to 6.
   The above-mentioned cyclic amine derivative in the nerve regeneration stimulator of the invention may be at least one selected from the group consisting of:
   1 -benzyl-4-((5,6-dimethoxy-1 -indanone)-2-yl)methylpiperidine,
   1-benzyl-4-((5,6-dimethoxy-1-indanone)-2-ylidenyl)methylpiperidine,
   1-benzyl-4-((5-methoxy-1-indanone)-2-yl)methylpiperidine,
   1-benzyl-4-((5,6-methylenedioxy-1-indanone)-2-yl)methylpiperidine,
   1-(m-nitrobenzyl)-4-((5,6-dimethoxy-1-indanone)-2-yl)methylpiperidine,
   1 -cyclohexylmethyl-4-((5,6-dimethoxy-1 -indanone)-2-yl)methylpiperidine,
   1 -(m-fluorobenzyl)-4-((5,6-dimethoxy-1 -indanone)-2-yl)methylpiperidine,
   1-benzyl-4-(3-((5,6-dimethoxy-1-indanone)-2-yl)propyl)piperidine,
   1-benzyl-4-((5-isopropoxy-6-methoxy-1-indanone)-2-yl)methylpiperidine,
   1-benzyl-4-((5,6-dimethoxy-1-indanone)-2-ylidenyl)propenylpiperidine, and
   1-benzyl-4-((5,6-dimethoxy-1,3-indandione)-2-yl)propenylpiperidine. Preferably, the cyclic amine derivative may be
   1-benzyl-4-((5,6-dimethoxy-1-indanone)-2-yl)methylpiperidine.

   The compound having an acetylcholinesterase inhibitory activity in the nerve regeneration stimulator of the invention is preferably
   1-benzyl-4-((5,6-dimethoxy-1-indanone)-2-yl)methylpiperidine hydrochloride and may be galantamine, tacrine, physostigmine or rivastigmine.
(2) A method for screening a substance that stimulates nerve regeneration, comprising: administering a test substance to neural stem cells, a tissue comprising neural stem cells or a non-human mammal; and detecting or determining a change in a phenotype for the nerve regeneration in the presence and absence of the candidate substance.
   The candidate substances include a compound having a cholinesterase inhibitory activity, a pharmacologically acceptable salt thereof or a solvate thereof. Preferably, the compound having a cholinesterase inhibitory activity is a compound with an acetylcholinesterase inhibitory activity, a pharmacologically acceptable salt thereof or a solvate thereof.
   The screening method of the invention may employ, as an index of the phenotype change for nerve regeneration, at least one selected from the group consisting of proliferation ability of cells derived from the neural stem cells, differentiation potential of the cells, shape of the cells, motility of the cells, migration ability and ability to regulate migration of the cells, maturity of the cells, expression level of the functional protein, shape of the dendrites, shape of the axon, shape of the synapse, ability to form synapse, survival of the cells, retention of the cells and control of cell death.
(3) A method for stimulating nerve regeneration, comprising administering an effective amount of a compound having the cholinesterase inhibitory activity described above, a pharmacologically acceptable salt thereof or a solvate thereof to a patient.

### BRIEF DESCRIPTION OF THE DRAWINGS

Figure 1 shows BrdU positive cells in the granule cell layer (GCL) of the hippocampal dentate gyrus. The black dots represent the nuclei showing positive immune reaction to BrdU. The number of BrdU positive cells in mice administered with donepezil increased as compared to mice administered with a vehicle.
Figure 2 shows expression of acetylcholine receptors (muscarine M2 receptors) (white arrows) on the BrdU positive cells present in the GCL of the hippocampal dentate gyrus.
Figure 3 shows the increase of intracellular calcium in the cultured rat neural progenitor cells. This shows that stimulation with cholinergic agents such as acetylcholine or carbachol increases the intracellular calcium concentration of the neural progenitor cells and that such action is completely inhibited with atropine (muscarine receptor antagonist).
Figure 4 shows expression of acetylcholine receptors (muscarine M2 receptors) (white arrows) of the cultured rat neural progenitor cells.

### BEST MODES FOR CARRYING OUT THE INVENTION

Hereinafter, embodiments of the present invention will be described. The following embodiments are merely meant to illustrate the present invention and the present invention is not limited thereto. The present invention may be carried out in various embodiments without departing from the scope of the invention.

All references, laid-open applications, patent publications and the like cited in this specification are herein incorporated by reference.

The present invention was accomplished by finding a mechanism that suppressing acetylcholine degradation with a cholinesterase (ChE) inhibitor increases the concentration of acetylcholine in the synaptic cleft, thereby maintaining the number of cells regenerating from the neural stem cells.

Thus, the present invention provides a nerve regeneration stimulator comprising, as an active element, a compound that suppresses acetylcholine degradation to increase acetylcholine concentration, namely, a compound having a cholinesterase inhibitory activity, a pharmacologically acceptable salt thereof or a solvate thereof, and a method for screening such drug.

### 1. Compound with cholinesterase (ChE) inhibitory activity

A nerve regeneration stimulator of the present invention comprises as an active element, a compound with a ChE inhibitory activity, a pharmacologically acceptable salt thereof or a solvate thereof. The compound with a ChE inhibitory activity according to the present invention refers to a substance with a ChE inhibitory activity, i.e., a substance that reversibly or irreversibly inhibits a ChE activity. In the present invention, ChE comprises acetylcholinesterase (AChE) (EC3.1.1.7), butyrylcholinesterase or the like. Preferable features of the compound with a ChE inhibitory activity of the present invention include that it is highly selective for AChE over butyrylcholinesterase, that it is capable of passing through the blood-brain barrier, and that it does not cause severe side effect at a dose required for treatment.

According to the present invention, a preferable compound used as a nerve regeneration stimulator comprises a compound with a ChE, particularly an AChE inhibitory activity. This compound comprises pharmacologically acceptable salts of a compound with a ChE inhibitory activity, solvates thereof and prodrugs thereof as described below.

### (1) Compound with cholinesterase inhibitory activity

According to the present invention, compounds with a ChE inhibitory activity include donepezil (ARICEPT^{®}), galantamine (Reminyl^{®}), tacrine (Cognex^{®}), rivastigmine (Exelon^{®}), zifrosilone (US Patent No.5693668 specification), physostigmine (Synapton) (Neurobiology of Aging 26 (2005) 939-946), ipidacrine (US Patent No.4550113 specification), quilostigmine, metrifonate (Promem) (US Patent No.4950658 specification), eptastigmine, velnacrine, tolserine, cymserine (US Patent No.6410747 specification), mestinon, icopezil (US Patent No.5750542 specification), TAK-147 (J.Med. Chem., 37(15), 2292-2299, 1994, Japanese Patent Publication No. 2650537, US Patent No.5273974 specification), huperzine A (Drugs Fut., 24, 647-663, 1999), stacofylline (US Patent No.4599338 specification), thiatolserine, neostigmine, eseroline or thiacymserine. The compound may also be a derivative or a prodrug of the above compounds. Alternatively, a pharmacologically acceptable salt thereof or a solvate of the above compounds, derivatives and prodrugs may also be included as preferred embodiments of the compound with a ChE inhibitory activity. The compound with a ChE inhibitory activity also includes the compound with a ChE inhibitory activity described in WO00/18391 pamphlet.

Galantamine and derivatives thereof are described in US Patent No.4663318 specification, WO88/08708 pamphlet, WO97/03987 pamphlet, US Patent No.6316439 specification, US Patent No.6323195 specification, US Patent No.6323196 specification and the like. Tacrine and derivatives thereof are described in US Patent No.4631286 specification, US Patent No.4695573 specification, US Patent No.4754050 specification, WO88/02256 pamphlet, US Patent No.4835275 specification, US Patent No.4839364 specification, US Patent No.4999430 specification, WO97/21681 pamphlet and the like. Physostigmine and derivatives thereof are described in US Patent No.5077289 specification, US Patent No.5177101 specification, US Patent No. 5302721 specification, Japanese Laid-Open Application No. 5-306286, US Patent No.7166824 specification, EP Patent No.298202 specification, WO98/27096 pamphlet, J. Pharm. Exp. Therap., 249 (1), 194-202, 1989 and the like. Rivastigmine and derivatives thereof are described in EP Patent No. 193926 specification, WO98/26775 pamphlet, WO98/27055 pamphlet and the like.

"Prodrug" as used herein means a drug obtained by chemically modifying "an active principle of a drug" (i.e., a "drug" corresponding to the prodrug) into an inactive element for the purpose of bioavailability improvement, alleviation of side effects or the like, which, after absorption, is metabolized to an active principle in the body and exerts action. Thus, the term "prodrug" refers to any compound that has a lower intrinsic activity than a corresponding "drug" but which, when administered to a biological system, generates the "drug" substance as a result of spontaneous chemical reaction, enzyme catalysis or metabolic reaction. Examples of such prodrugs include those in which an amino group, a hydroxyl group or a carboxyl group of the above-exemplified compound or a compound represented by the general formula below has been acylated, alkylated, phosphorylated, borated, carbonated, esterified, amidated or urethanated. This exemplified group, however, merely represents typical examples and thus is not comprehensive. Those skilled in the art can prepare other various known prodrugs from the above-exemplified compound or the compound represented by the general formula below according to a known method. A prodrug comprising the above-exemplified compound or the compound represented by the general formula below is within the scope of the invention.

### (2) Cyclic amine derivatives

According to the present invention, preferred examples of a compound with a ChE inhibitory activity, specifically an AChE inhibitory activity further include a cyclic amine derivative represented by the following general formula (I), a pharmacologically acceptable salt and a solvate thereof. According to the present invention, a compound with a ChE inhibitory activity is preferably 1-benzyl-4-[(5,6-dimethoxy-1-indanone)-2-yl]methylpiperidine (donepezil), a pharmacologically acceptable salt thereof or a solvate thereof, more preferably 1-benzyl-4-[(5,6-dimethoxy-1-indanone)-2-yl] methylpiperidine hydrochloride (donepezil hydrochloride), i.e., ARICEPT^{®}. 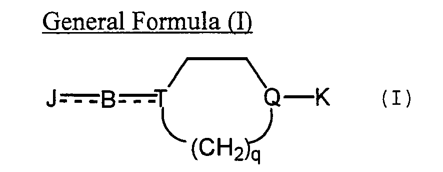 (wherein, J refers to one selected from groups (a) to (e) listed below:
(a) a substituted or unsubstituted
   (1) phenyl group,
   (2) pyridyl group,
   (3) pyradyl group,
   (4) quinolyl group,
   (5) cyclohexyl group,
   (6) quinoxalyl group or
   (7) furyl group;
(b) a monovalent or divalent group derived from one selected from the group consisting of
   (1) indanyl,
   (2) indanonyl,
   (3) indenyl,
   (4) indenonyl,
   (5) indandionyl,
   (6) tetralonyl,
   (7) benzsuberonyl,
   (8) indanolyl and
   (9) a group represented by formula 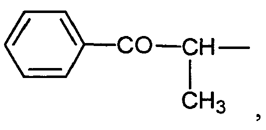 in all of which a phenyl group may be substituted,
(c) a monovalent group derived from a cyclic amide compound,
(d) a lower alkyl group, or
(e) a group represented by formula R¹-CH=CH- (wherein, R¹ is a hydrogen atom or a lower alkoxycarbonyl group),
   B refers to a group represented by formula 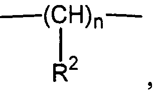 a group represented by formula 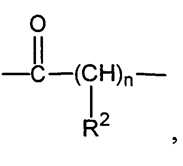 a group represented by formula 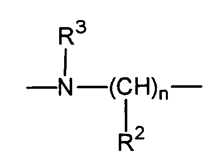 (wherein, R³ is a hydrogen atom, a lower alkyl group, an acyl group, a lower alkylsulfonyl group, a substituted or unsubstituted phenyl group or a benzyl group), a group represented by formula 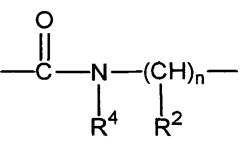 (wherein, R⁴ is a hydrogen atom, a lower alkyl group or a phenyl group), a group represented by formula 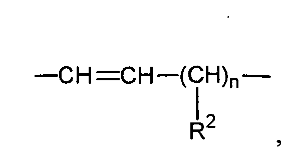 a group represented by formula 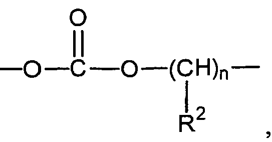 a group represented by formula 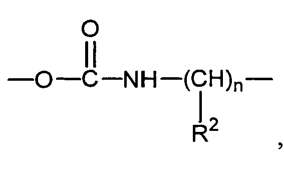 a group represented by formula 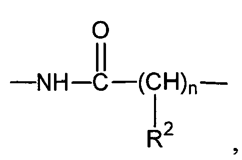 a group represented by formula 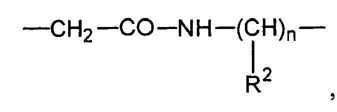 a group represented by formula 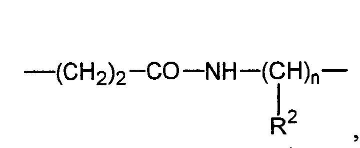 a group represented by formula 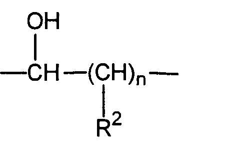 (wherein, n is 0 or an integer of 1 to 10, and R² is a hydrogen atom or a methyl group), a group represented by formula =(CH-CH=CH)_{b} - (wherein, b is an integer of 1 to 3), a group represented by formula =CH-(CH₂)_{c}- (wherein, c is 0 or an integer of 1 to 9), a group represented by formula =(CH-CH)_{d}= (wherein, d is 0 or an integer of 1 to 5), a group represented by formula 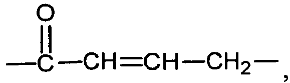 a group represented by formula 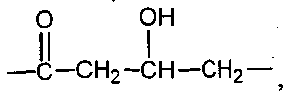 a group represented by formula 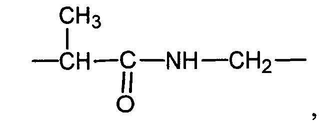 a group represented by formula 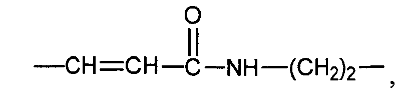 a group represented by formula -NH-, a group represented by formula -O-, a group represented by formula -S-, a dialkylaminoalkylcarbonyl group or a lower alkoxycarbonyl group,
   T represents a nitrogen atom or a carbon atom,
   Q represents a nitrogen atom, a carbon atom or a group represented by formula 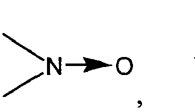 K is a hydrogen atom, a substituted or unsubstituted phenyl group, an arylalkyl group in which a phenyl group may be substituted, a cinnamyl group in which a phenyl group may be substituted, a lower alkyl group, a pyridylmethyl group, a cycloalkylalkyl group, an adamantanemethyl group, a furylmethyl group, a substituted or unsubstituted cycloalkyl group, a lower alkoxycarbonyl group or an acyl group,
   q is an integer of 1 to 3, and
   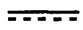 indicates a single bond or a double bond).

"A lower alkyl group" as used herein comprises a straight or branched alkyl group with a carbon number 1 to 6, for example, a methyl group, an ethyl group, an n-propyl group, an isopropyl group, an n-butyl group, an isobutyl group, a sec-butyl group, a tert-butyl group, a pentyl group (an amyl group), an isopentyl group, a neopentyl group, a tert-pentyl group, a 1-methylbutyl group, a 2-methylbutyl group, a 1,2-dimethylpropyl group, a hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group and the like. Preferable groups among them include a methyl group, an ethyl group, a propyl group and an isopropyl group, most preferable group being a methyl group. "A lower alkyl group" is described in the definition of the above compound (I) of the present invention, for example, in the definitions of J, K, R³ and R⁴.

"A lower alkoxy group" as used herein means a lower alkoxy group corresponding to the above-mentioned lower alkyl group such as a methoxy group and an ethoxy group.

"A lower alkoxycarbonyl group" as used herein means a lower alkoxycarbonyl group corresponding to the above-mentioned lower alkoxy group such as a methoxycarbonyl group, an ethoxycarbonyl group, an isopropoxycarbonyl group, an n-propoxycarbonyl group and an n-butyloxycarbonyl group.

"A cycloalkyl group" as used herein refers to a cyclic alkyl group with a carbon number 4 to 10, including but not limited to a cyclobutyl group, a cyclopentyl group and a cyclohexyl group.

### "J"

In the definition of J, exemplary substituents for "(a) substituted or unsubstituted (1) phenyl group, (2) pyridyl group, (3) pyradyl group, (4) quinolyl group, (5) cyclohexyl group, (6) quinoxalyl group or (7) furyl group" include:
a lower alkyl group with a carbon number 1 to 6 such as a methyl group, an ethyl group, an n-propyl group, an isopropyl group, an n-butyl group, an isobutyl group and a tert-butyl group;
a lower alkoxy group corresponding to a lower alkyl group such as a methoxy group and an ethoxy group;
a nitro group;
a halogen such as chlorine, bromine and fluorine;
a carboxyl group;
a lower alkoxycarbonyl group corresponding to the lower alkoxy group above such as a methoxycarbonyl group, an ethoxycarbonyl group, an isopropoxycarbonyl group, an n-propoxycarbonyl group and an n-butyloxycarbonyl group;
an amino group;
a mono-lower alkylamino group;
a di-lower alkylamino group;
a carbamoyl group;
an acylamino group derived from aliphatic saturated monocarboxylic acid with a carbon number 1 to 6 such as an acetylamino group, a propionylamino group, a butyrylamino group, an isobutyrylamino group, a valerylamino group and a pivaloyl amino group;
a cycloalkyloxycarbonyl group such as a cyclohexyloxycarbonyl group;
a lower alkylaminocarbonyl group such as a methylaminocarbonyl group and an ethylaminocarbonyl group;
a lower alkylcarbonyloxy group corresponding to the lower alkyl group defined above such as a methylcarbonyloxy group, an ethylcarbonyloxy group and an n-propylcarbonyloxy group;
a halogenated lower alkyl group as represented by a trifluoromethyl group or the like;
a hydroxyl group;
a formyl group; and
a lower alkoxy lower alkyl group such as an ethoxymethyl group, a methoxymethyl group and a methoxyethyl group. As to the above substituents, "the lower alkyl group" and "the lower alkoxy group" comprise all of the groups that can be derived from the definition described above. Groups (1) to (7) in set (a) may be substituted with 1 to 3 of the same or different substituents mentioned above.

In the case of the phenyl group, the following case is also to be included in the substituted phenyl group: specifically, when the group can be represented by formula 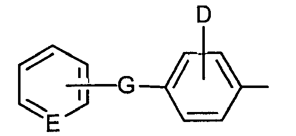 (wherein, G is a group represented by formula 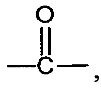 a group represented by formula 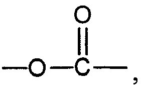 a group represented by formula -O-, a group represented by formula 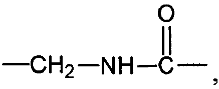 a group represented by formula -CH₂-O-, a group represented by formula -CH₂-SO₂-, a group represented by formula 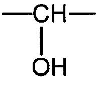 or a group represented by formula 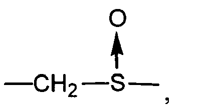 E represents a carbon atom or a nitrogen atom),
D may represent a lower alkyl group with a carbon number 1 to 6 such as a methyl group, an ethyl group, an n-propyl group, an isopropyl group, an n-butyl group, an isobutyl group, and a tert-butyl group;
a lower alkoxy group corresponding to the lower alkyl group above such as a methoxy group and an ethoxy group;
a nitro group;
a halogen such as chlorine, bromine and fluorine;
a carboxyl group;
a lower alkoxycarbonyl group corresponding to the lower alkoxy group above such as a methoxycarbonyl group, an ethoxycarbonyl group, an isopropoxycarbonyl group, an n-propoxycarbonyl group and an n-butyloxycarbonyl group;
an amino group;
a mono-lower amino group;
a di-lower alkylamino group;
a carbamoyl group;
an acylamino group derived from aliphatic saturated monocarboxylic acid with a carbon number 1 to 6 such as an acetylamino group, a propionylamino group, a butyrylamino group, an isobutyrylamino group, a valerylamino group and a pivaloylamino group;
a cycloalkyloxycarbonyl group such as a cyclohexyloxycarbonyl group;
a lower alkylaminocarbonyl group such as a methylaminocarbonyl group and an ethylaminocarbonyl group;
a lower alkylcarbonyloxy group corresponding to the lower alkyl group defined above such as a methylcarbonyloxy group, an ethylcarbonyloxy group and an n-propylcarbonyloxy group;
a halogenated lower alkyl group as represented by a trifluoromethyl group;
a hydroxyl group;
a formyl group;
a lower alkoxy lower alkyl group such as an ethoxymethyl group, a methoxymethyl group and a methoxyethyl group. As to the substituents described above, "the lower alkyl group" and "the lower alkoxy group" comprise all of the groups that can be derived from the definition described above).

Among those mentioned above, substituents favorable for a phenyl group include a lower alkyl group, a lower alkoxy group, a nitro group, a halogenated lower alkyl group, a lower alkoxycarbonyl group, a formyl group, a hydroxyl group, a lower alkoxy lower alkyl group, a halogen, a benzoyl group and a benzylsulfonyl group. The substituents may be two or more and may be the same or different.

Preferred substituents for a pyridyl group may include a lower alkyl group, an amino group and a halogen atom.

Preferred substituents for a pyradyl group may include a lower alkoxycarbonyl group, a carboxyl group, an acylamino group, a carbamoyl group and a cycloalkyloxycarbonyl group.

When representing "J", 2-pyridyl group, 3-pyridyl group or 4-pyridyl group is desirable as a pyridyl group, 2-pyradyl group is desirable as a pyradyl group, 2-quinolyl group or 3-quinolyl group is desirable as a quinolyl group, 2-quinoxalyl group or 3-quinoxalyl group is desirable as a quinoxalyl group, and 2-furyl group is desirable as a furyl group.

In the definition of "J", typical examples of the monovalent or divalent group derived from (1) to (9) listed in set (b) are shown below:

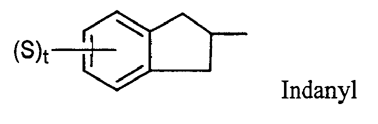

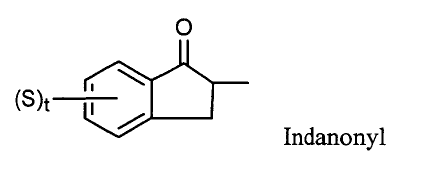

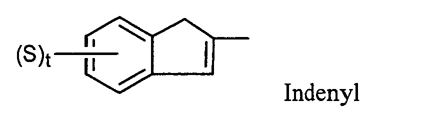

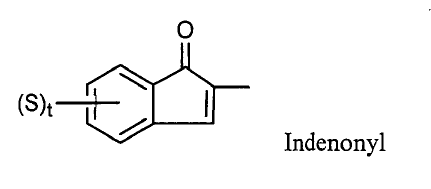

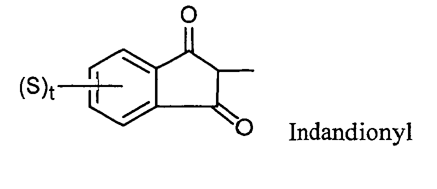

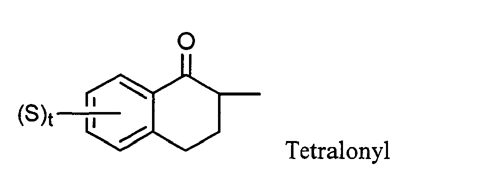

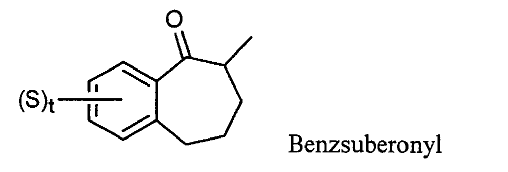

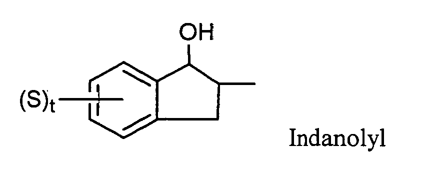

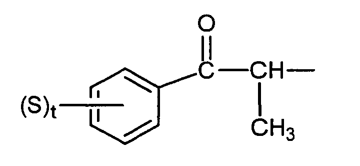

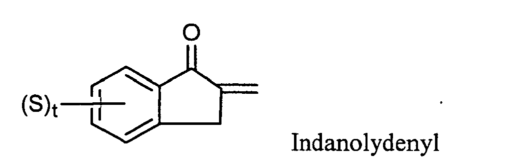

In the above series of formulae, t means 0 or an integer of 1 to 4, indicating that the phenyl group is substituted by 0, or 1 to 4 same or different groups indicated by S. S identically or differently indicates one of the substituents listed in set (a) in the definition of J or a hydrogen atom and preferably includes a hydrogen atom (unsubstituted), a lower alkyl group or a lower alkoxy group. Furthermore, the phenyl group may be substituted by an alkylenedioxy group such as a methylenedioxy group or an ethylenedioxy group between adjacent carbons of the phenyl ring.

Among those mentioned above, preferably, no substitution exists, 1 to 3 methoxy groups or isopropoxy groups are substituted, or a methylenedioxy group is substituted. Most preferably, no substitution exists, or 1 to 3 methoxy groups are substituted.

The above-mentioned indanolydenyl is an example of a divalent group in which a phenyl group listed in (b) in the definition of J may be substituted, i.e., a typical divalent group derived from (2) indanonyl in J (b).

In the definition of J, examples of the monovalent group derived from a cyclic amide compound from (c) include, for example, quinazolone, tetrahydroisoquinoline-one, tetrahydrobenzodiazepine-one and hexahydrobenzazocin-one, but are not limited thereto as long as a cyclic amide exists in the structural formula.

The cyclic amide may be derived from a monocyclic ring or a condensed heterocyclic ring. Preferably, the condensed heterocyclic ring is a condensed heterocyclic ring with a phenyl ring. In this case, the phenyl ring may be substituted with a lower alkyl group with a carbon number 1 to 6, preferably a methyl group, a lower alkoxy group with a carbon number 1 to 6, preferably a methoxy group or a halogen atom.

Preferable examples include the following: 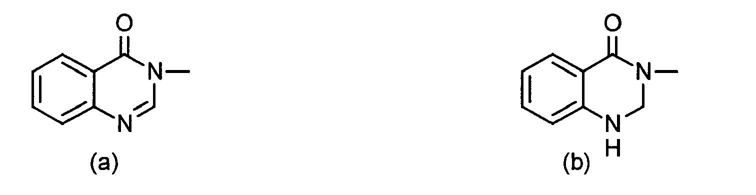 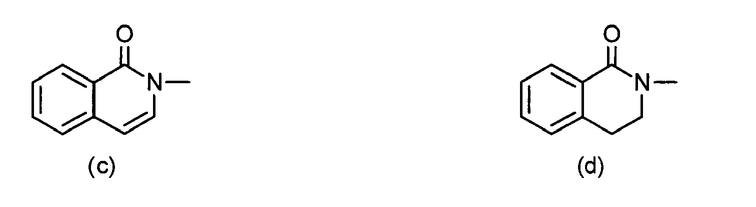 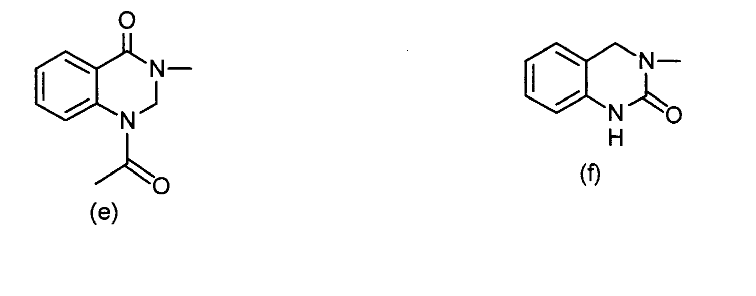 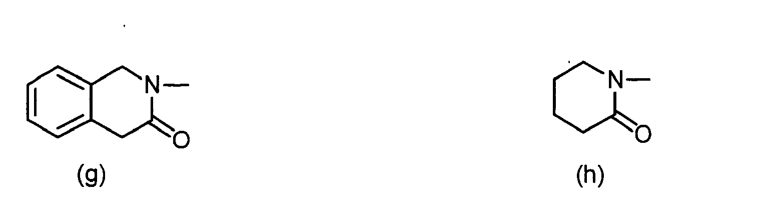 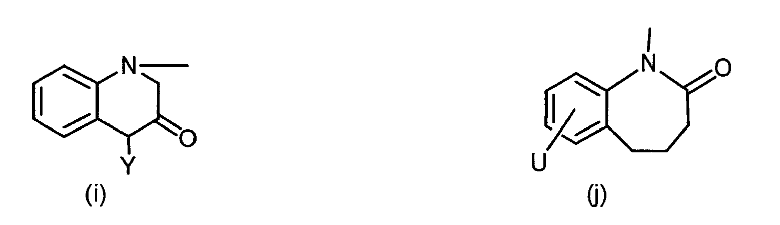 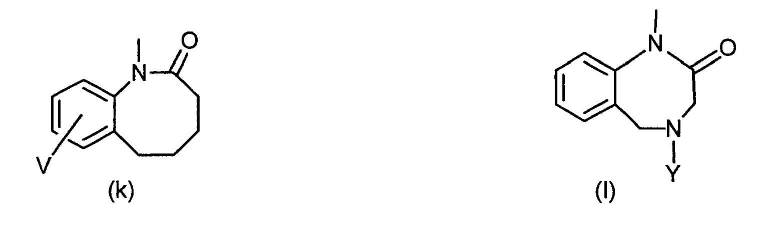 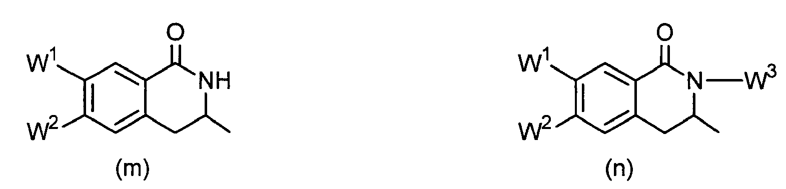 (wherein, Y in formulae (i) and (1) represents a hydrogen atom or a lower alkyl group, V in formula (k) represents a hydrogen atom or a lower alkoxy group, W¹ and W² in formulae (m) and (n) represent a hydrogen atom, a lower alkyl group or a lower alkoxy group, and W³ represents a hydrogen atom or a lower alkyl group. U in formula (j) represents a hydrogen atom, a lower alkyl group or a lower alkoxy group.

The rings on the right side in formulae (j) and (1) are seven-membered rings, and the ring on the right side in formula (k) is an eight-membered ring.

For the definition of J, "(d) a lower alkyl group" is as described above.

Among those included in the above definition of J, groups included in sets (a) to (c) are preferable, most preferable group being a monovalent group derived from indanone (indanonyl) included in (b) where a phenyl ring may be substituted or unsubstituted, and a monovalent group derived from a cyclic amide compound included in (c).

### "B"

For the definition of B, a group represented by formula: 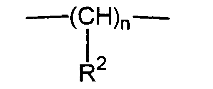 is indicated as formula -(CH₂)ₙ- when R² is a hydrogen atom. In this case, any of the carbon atoms of the alkylene chain may further bind to one or more methyl groups and n is preferably 1 to 3.

In B, examples of "dialkylaminoalkylcarbonyl group" include, for example, N,N-dimethylaminoalkyl carbonyl group, N,N-diethylaminoalkyl carbonyl group, N,N-diisopropylaminoalkyl carbonyl group, and N-methyl-N-ethylaminoalkyl carbonyl group.

As to a series of groups of B, a group including an amide group is also preferable.

Examples of preferable groups further include a group represented by formula -CH=CH-(CH)ₙR²- (wherein, n is 0 or an integer of 1 to 10, and R² is a hydrogen atom or a methyl group), a group represented by formula =(CH-CH=CH)_{b}- (wherein, b is an integer of 1 to 3), a group represented by formula =CH-(CH₂)_{c}- (wherein, c is 0 or an integer of 1 to 9), a group represented by formula =(CH-CH)_{d}= (wherein, d represents 0 or an integer of 1 to 5), a group represented by formula -NH-, a group represented by formula -O- and a group represented by formula -S-.

### "T", "Q" and "q"

A ring 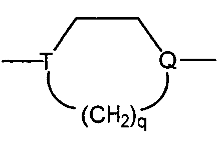 may be a five- to seven-membered ring. Specifically, examples of such ring include 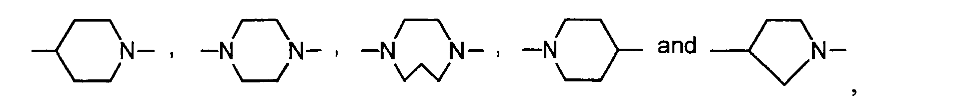 although particularly preferable ring is piperidine represented by formula

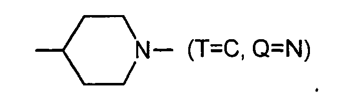

### "K " and "bonds"

As to expressions "substituted or unsubstituted phenyl group", "substituted or unsubstituted arylalkyl group (where a phenyl group may be substituted)", "cinnamyl group where a phenyl group may be substituted" and "cycloalkyl group which may be substituted" in the definition of K, the substituents are the same as those defined in definition of J for (a) (1) to (7). These are preferably unsubstituted or may be substituted with a nitro group, a lower alkyl group such as methyl or a halogen such as fluorine.

An arylalkyl group is intended to mean a benzyl group or a phenetyl group in which a phenyl ring is unsubstituted or substituted with a substituent described above.

Examples of pyridylmethyl group may specifically include 2-pyridylmethyl group, 3-pyridylmethyl group and 4-pyridylmethyl group.

Most preferably, K is an arylalkyl group where a phenyl group may be substituted, a substituted or unsubstituted phenyl group, a cinnamyl group where a phenyl group may be substituted and a cycloalkyl group that may be substituted.

Preferable arylalkyl group is specifically, for example, a benzyl group or a phenetyl group in which a phenyl group may be substituted with a lower alkoxy group having a carbon number 1 to 6, a lower alkyl group having a carbon number 1 to 6, a hydroxyl group or the like.

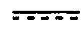 indicates a single bond or a double bond.

An exemplary case of the double bond includes the above-described divalent group derived from indanone where a phenyl ring may be substituted, namely an indanolydenyl group.

### Compound set (A)

Gathering from these definitions, particularly preferable compound set include compound set (A) represented by the following general formula, i.e., a cyclic amine represented by formula: 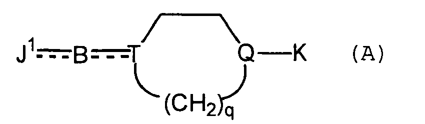 (wherein, J¹ is a monovalent or divalent group derived from a group selected from the group consisting of:
(1) indanyl,
(2) indanonyl,
(3) indenyl,
(4) indenonyl,
(5) indandionyl,
(6) tetralonyl,
(7) benzsuberonyl,
(8) indanolyl, and
(9) a group represented by formula 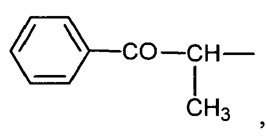 in all of which a phenyl group may be substituted; and
   B, T, Q, q, K and 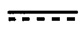 have the same meaning as described above), a pharmacologically acceptable salt thereof or a solvate thereof.

In the above definition of J¹, the most preferable groups include an indanonyl group, an indandionyl group and an indanolydenyl group where a phenyl group may be substituted. Most preferably, a phenyl group is unsubstituted or substituted identically or differently with a hydroxyl group, a halogen or a lower alkoxy group, or substituted with an alkylenedioxy group between adjacent carbon atoms of a phenyl ring. A lower alkoxy group refers to, for example, a methoxy group, an ethoxy group, an isopropoxy group, an n-propoxy group and an n-butoxy group with a carbon number 1 to 6, and can take a form of mono- to tetra-substitution, preferably disubstitution. Most preferably, the methoxy group takes a form of disubstitution.

### Compound set (B)

More preferable compound set included in the compound set of formula (A) may include a compound set represented by the following general formula (B): 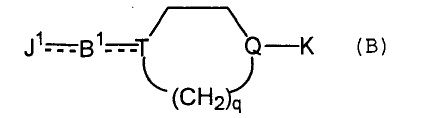 (wherein, J¹ is the same as described above,
B¹ is a group represented by 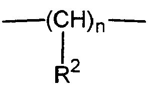 (wherein, n is 0 or an integer of 1 to 10, and R² is a hydrogen atom or a methyl group), a group represented by formula -CH=CH-(CH)ₙR²- (wherein, n represents 0 or an integer of 1 to 10, and R² represents a hydrogen atom or a methyl group), a group represented by formula =(CH-CH=CH)_{b}-(wherein, b is an integer of 1 to 3), a group represented by formula =CH-(CH₂)_{c}-(wherein, c represents 0 or an integer of 1 to 9) or a group represented by formula =(CH-CH)_{d}= (wherein, d is 0 or an integer of 1 to 5): Preferably, B¹ is a group represented by formula -(CH)ₙR²- (wherein, n is 0 or an integer of 1 to 10, and R² is a hydrogen atom or a methyl group), more preferably -CH₂- (n = 1, and R² is a hydrogen atom), or -CH₂-CH₂-CH₂- (n = 3, and R² is a hydrogen atom): B¹ is preferably a group represented by formula -CH=CH-(CH)ₙR²- (wherein, n is 0 or an integer of 1 to 10, and R² is a hydrogen atom or a methyl group), more preferably -CH=CH-CH₂- (n = 1 and R² is a hydrogen atom), and
T, Q, q, K and 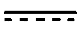 are as described above).

### Compound set (C)

More preferable compound set included in the compound set of formula (B) may include a compound set represented by the following general formula (C): 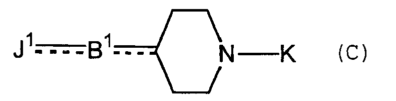 (wherein, J¹, B¹, K and 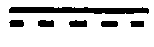 are as described above).

Specifically, the group represented by formula 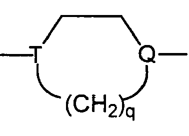 iS indicated by a group represented by formula 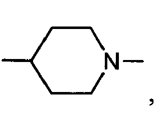 i.e., piperidine.

### Compound set (D)

More preferable compound set included in the compound set of formula (C) may include a compound set represented by the following general formula (D): 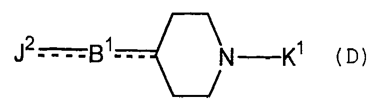 (wherein, J² is a group selected from a monovalent or divalent group derived from indanonyl where a phenyl group may be substituted (e.g., indanonyl, indanolydenyl group), indenyl and indandionyl: More preferably, J² is an indanonyl group which may have, as a substituent, a lower alkyl group with a carbon number 1 to 6 or a lower alkoxy group with a carbon number 1 to 6,
K¹ is a substituted or unsubstituted phenyl group, an arylalkyl group which may be substituted, a cinnamyl group which may be substituted or a cycloalkyl group which may be substituted, and
B¹ and 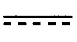 are as described above).

According to the present invention, a preferable compound having a ChE inhibitory activity includes the following:
1-benzyl-4-((5,6-dimethoxy-1-indanone)-2-yl)methylpiperidine,
1-benzyl-4-((5,6-dimethoxy-1-indanone)-2-ylidenyl)methylpiperidine,
1-benzyl-4-((5-methoxy-1-indanone)-2-yl)methylpiperidine,
1-benzyl-4-((5,6-methylenedioxy-1-indanone)-2-yl)methylpiperidine,
1-(m-nitrobenzyl)-4-((5,6-dimethoxy-1-indanone)-2-yl)methylpiperidine,
1-cyclohexylmethyl-4-((5,6-dimethoxy-1-indanone)-2-yl)methylpiperidine,
1-(m-fluorobenzyl)-4-((5,6-dimethoxy-1-indanone)-2-yl)methylpiperidine,
1-benzyl-4-(3 -((5,6-dimethoky-1-indanone)-2-yl)propyl)piperidine,
1-benzyl-4-((5-isopropoxy-6-methoxy-1-indanone)-2-yl)methylpiperidine,
1-benzyl-4-((5,6-dimethoxy-1-indanone)-2-ylidenyl)propenylpiperidine, and
1 -benzyl-4-((5,6-dimethoxy-1,3-indandione)-2-yl)propenylpiperidine, more preferably 1-benzyl-4-((5,6-dimethoxy-1-indanone)-2-yl)methylpiperidine.

### (3) Production process

A compound having a ChE inhibitory activity, a pharmacologically acceptable salt thereof or a solvate thereof used in the present invention can be produced according to a known method. The cyclic amine derivatives represented by the general formula (I) above (e.g., donepezil hydrochloride) can readily be produced by methods disclosed, as representative examples, in Japanese Laid-Open Application No. 1-79151, Japanese Patent Publication No. 2578475, Japanese Patent Publication No. 2733203, Japanese Patent Publication No. 3078244 or US Patent Publication No.4895841. Donepezil hydrochloride is also available as formulations such as fine granules.

Galantamine and derivatives thereof can readily be produced by methods disclosed in US Patent No.4663318 specification, WO88/08708 pamphlet, WO97/03987 pamphlet, US Patent No.6316439 specification, US Patent No.6323195 specification and US Patent No.6323196 specification.

Tacrine and derivatives thereof can readily be produced by methods disclosed in US Patent No.4631286 specification, US Patent No.4695573 specification, US Patent No.4754050 specification, WO88/02256 pamphlet, US Patent No.4835275 specification, US Patent No.4839364 specification, US Patent No.4999430 specification, and WO97/21681 pamphlet.

Physostigmine and derivatives thereof can readily be produced by methods disclosed in US Patent No. 5077289 specification, US Patent No. 5177101 specification, US Patent No. 5302721 specification, Japanese Laid-Open Application No.5-306286, US Patent No.7166824 specification, EP Patent No. 298202 specification, WO98/27096 pamphlet, and J. Pharm. Exp. Therap., 249 (1), 194 - 202, 1989.

Rivastigmine and derivatives thereof can readily be produced by methods disclosed, for example, in EP Patent No. 193926 specification, WO98/26775 pamphlet, and WO 98/27055 pamphlet.

Among these compounds, those that are commercially available can readily be obtained from, for example, chemical manufacturers.

According to the present invention, examples of pharmacologically acceptable salts include, for example, inorganic acid salts such as hydrochloride, sulfate, hydrobromate and phosphate, or organic acid salts such as formate, acetate, trifluoroacetate, maleate, tartrate, methanesulfonate, benzenesulfonate and toluenesulfonate.

In addition, depending on the choice of the substituent, for example, alkali metal salts such as sodium salt and potassium salt, alkaline earth metal salts such as calcium salt and magnesium salt, organic amine salts such as trimethylamine salt, triethylamine salt, pyridine salt, picoline salt, dicyclohexylamine salt and N,N'-dibenzylethylenediamine salt, or ammonium salt may be formed.

A compound having a ChE inhibitory activity or a pharmacologically acceptable salt thereof (e.g., donepezil hydrochloride) as an active element for a nerve regeneration stimulator may be an anhydride, and may form a solvate such as a hydrate. According to the present invention, a solvate is preferably a pharmacologically acceptable solvate. A pharmacologically acceptable solvate may be either a hydrate or a nonhydrate, but preferably a hydrate. A solvent such as water, alcohol (e.g., methanol, ethanol, n-propanol), dimethylformamide, dimethyl sulfoxide (DMSO) or the like may be used.

According to the present invention, the above-mentioned compound may have an asymmetric carbon depending on the type of substituent and may have an enantiomer, which are within the scope of the present invention. Furthermore, crystal polymorph may exist in the above-mentioned compound (e.g., donepezil), although not limited thereto and any form of crystal may exist alone or in combination, which are within the scope of the present invention.

In one specific example, if J has an indanone skeleton associated with an asymmetric carbon, a geometric isomer, an enantiomer, a diastereomer or the like may exist. All of these cases are within the scope of the present invention.

### 2. Nerve regeneration stimulator

A nerve regeneration stimulator of the present invention refers to a drug that affects the process where stem cells (e.g., neural stem cells, embryonic stem cells, etc.) from human or organisms other than human (e.g., non-human mammals including cow, monkey, avian, cat, mouse, rat, guinea pig, hamster, pig, dog and rabbit) differentiate into neural progenitor cells, which are, in turn, incorporated into the neural circuit as functionally mature nerve cells and thereby stimulates nerve regeneration. According to the present invention, "nerve regeneration" refers to one that is based on at least one of the following action mechanisms: promoting neural stem cells to self-renew, promoting neural stem cells to proliferate, promoting neural stem cells to differentiate into neural progenitor cells, promoting neural progenitor cells to proliferate, promoting neural progenitor cells to mature into nerve cells, promoting neural stem cells or neural progenitor cells to survive, inhibiting cell death of neural stem cells or neural progenitor cells and promoting synapse formation of regenerated nerve cells.

The compound having a ChE inhibitory activity described above, a pharmacologically acceptable salt thereof or a solvate thereof stimulates nerve regeneration. In addition, they are also useful as an active element of a therapeutic agent for diseases that damage nerve function of the hippocampus.

Thus, the present invention also provides a method for stimulating nerve regeneration, comprising administering an effective amount of the compound of the invention having a ChE inhibitory activity, a pharmacologically acceptable salt thereof or a solvate thereof to a patient.

The nerve regeneration stimulator of the invention stimulates nerve regeneration and thus can be used for treating social anxiety disorders.

One possible cause of social anxiety disorders is said to be a decrease in nerve regeneration in hippocampus due to stress or trauma. The following (i) to (v) suggest that SSRIs possibly have a treatment effect via stimulation of nerve regeneration in the hippocampus of PTSD patients.
(i) Reduced volume of the hippocampus in patients suffering from post-traumatic stress disorder (PTSD) has been reported. This reduction of the hippocampus volume is said to be one of the factors that develop social anxiety disorders such as PTSD.
(ii) The nerve regeneration in the hippocampus is decreased in animal models of social anxiety disorder.
(iii) Based on the results from the animal experiments of (ii), decrease in the nerve regeneration in the hippocampus is said to be one reason of (i).
(iv) Administration of SSRI (paroxetine) used for treating social anxiety disorders is reported to increase the hippocampal volume of PTSD patients.
(v) SSRI is reported to stimulate nerve regeneration in hippocampus.

If SSRIs show treatment effects on social anxiety disorders via a nerve regeneration stimulating action, a drug that stimulates nerve regeneration has a possibility to be used as an agent for treating social anxiety disorders. Thus, (i) to (v) described above show that the nerve regeneration stimulator of the invention is effective as an agent for treating social anxiety disorders. Social anxiety disorder is appropriate as a target of the nerve regeneration stimulating agents of the invention, i.e., the nerve regeneration stimulating agents of the invention may be used for treating social anxiety disorders.

The term "treatment" generally means an achievement of a desirable pharmacological effect and/or physiological effect. These effects can be prophylactic in terms of completely or partially preventing a disease and/or symptoms, and therapeutic in terms of partially or completely curing a disease and/or adverse effects caused by a disease. Herein, "treatment" refers to any treatment for a disease of a mammal, particularly human, and also includes general treatment as described above. "Treatment" includes, for example, the following (a) to (c):
(a) to prevent a disease or a symptom in a patient who is predisposed to the disease or the symptom but not yet diagnosed to be so;
(b) to inhibit a disease or a symptom, that is, to stop or delay the progress thereof;
(c) to alleviate a disease or a symptom, that is, to delay or eliminate the disease or the symptom, or to reverse the progress of the symptom.

A compound with a ChE inhibitory activity, a salt thereof or a solvate thereof, or a prodrug thereof, a salt thereof or a solvate thereof may be administered either orally or parenterally to a human or non-human mammal (e.g., intravenous injection, muscle injection, subcutaneous injection, rectal administration, transdermal administration) by any one of various means. A compound having a ChE inhibitory activity, a salt thereof or a solvate thereof, or a prodrug thereof, a salt thereof or a solvate thereof may be used alone or may be formulated into an appropriate formulation using a pharmaceutical carrier by employing a conventionally used method depending on the administration route.

Examples of preferable formulations include, for example, oral formulations such as tablets, powder, fine granules, granules, coated tablets, capsules, syrup and lozenge, and parenteral formulations such as inhalers, suppositories, injectable agents (including intravenous fluids), ointments, ophthalmic drops, ophthalmic ointments, nasal drops, ear drops, adhesive patches, skin pads, lotion and liposome formulations.

Examples of carriers that can be used for formulating these formulations include, for example, a generally used solvent, excipient, coating agent, binder, disintegrating agent, lubricant, colorant, flavoring or aromatic substance, and if necessary, a stabilizer, an emulsifying agent, an absorption promoter, a surfactant, a pH regulator, an antiseptics, an antioxidant, a filler, a wetting agent, a surface-active agent, a dispersant, a buffer, a preservative, a solubilizing agent, a suspending agent, a thickening agent, a soothing agent and a tonicity agent, which can be formulated according to a common procedure by blending materials generally used for formulating a medicinal formulation. Examples of such non-toxic materials available include, for example, animal and vegetable oils such as soybean oil, beef tallow and synthetic glyceride; for example, hydrocarbons such as liquid paraffin, squalane and solid paraffin; for example, ester oils such as octyldodecyl myristate and isopropyl myristate; for example, higher alcohols such as cetostearyl alcohol and behenyl alcohol; silicon resin; silicon oil; for example, surfactants such as polyoxyethylene fatty acid ester, sorbitan fatty acid ester, glycerine fatty acid ester, polyoxyethylene sorbitan fatty acid ester, polyoxyethylene hardened caster oil and polyoxyethylene-polyoxypropylene block copolymer; for example, water-soluble polymers such as hydroxyethylcellulose, polyacrylic acid, carboxy vinyl polymer, polyethylene glycol, polyvinyl pyrrolidone and methylcellulose; for example, lower alcohols such as ethanol and isopropanol; for example, polyol such as glycerine, propylene glycol, dipropylene glycol, sorbitol and polyethylene glycol; for example, saccharides such as glucose and sucrose; for example, inorganic powers such as anhydrous silicon, magnesium aluminum silicate and aluminum silicate; inorganic salts such as sodium chloride and sodium phosphate; and purified water.

Examples of excipients include, for example, lactose, fructose, cornstarch, white sugar, glucose, mannitol, sorbit, crystalline cellulose and silicon dioxide; examples of binders include, for example, polyvinyl alcohol, polyvinyl ether, methylcellulose, ethylcellulose, gum arabic, tragacanth, gelatin, shellac, hydroxypropylmethylcellulose, hydroxypropylcellulose, polyvinyl pyrrolidone, polypropyleneglycol polyoxyethylene block copolymer and meglumine; examples of disintegrating agents include, for example, starch, agar, gelatin powder, crystalline cellulose, calcium carbonate, sodium hydrogen carbonate, calcium citrate, dextrin, pectin and carboxymethylcellulose calcium; examples of lubricants include, for example, magnesium stearate, talc, polyethylene glycol, silica and hardened plant oil; examples of colorants include pharmaceutically acceptable additives; and examples of flavoring or aromatic substances include cocoa powder, menthol, aromatic powder, mint oil, borneol and cinnamon powder. The materials mentioned above may be salts or solvates thereof.

An oral formulation is produced, for example, into powder, fine granule, granule, a tablet, a coated tablet, a capsule or the like according to a routine procedure after adding an excipient, and if necessary, further a binder, a disintegrating agent, a lubricant, a colorant, a flavoring or aromatic substance or the like to a compound having a ChE inhibitory activity, a salt thereof or a solvate thereof, or a prodrug thereof, a salt thereof or a solvate thereof.

Tablets and granules may be coated according to a well-known method using a coating agent such as carnauba wax, hydroxypropylmethylcellulose, macrogol, hydroxypropylmethyl phthalate, cellulose acetate phthalate, white sugar, titanium oxide, sorbitan fatty acid ester or calcium phosphate.

Specific examples of carrier used for producing a syrup agent include sweetening agents such as white sugar, glucose and fructose, suspending agents such as gum arabic, tragacanth, carmellose sodium, methylcellulose, sodium alginate, crystalline cellulose and veegum, and dispersants such as sorbitan fatty acid ester, sodium lauryl sulphate and polysorbate 80. For production of syrup, a flavoring material, an aromatic material, a preservative, a solubilizing agent and a stabilizer can be added as may be necessary. The product may be in a form of dry syrup that can be dissolved or suspended upon use.

An injectable agent is generally prepared by dissolving, for example, a salt of a compound having a ChE inhibitory activity in injectable distilled water, and may be formulated according to a common procedure by adding a solubilizing agent, a buffer, a pH regulator, a tonicity agent, a soothing agent, a preservative, a stabilizer or the like as may be necessary. The injectable agent may be asepticized by filter sterilization using a filter or by blending a disinfectant. The injectable agent may be produced into a form that can be prepared upon use. Specifically, the injectable agent may be prepared into a sterile solid composition by lyophilization or the like which can be dissolved in sterile injectable distilled water or other solvent before use.

Production of an external medicine is not limited to a particular production procedure and may be produced by any routine procedure. Various materials generally used in pharmaceuticals, medicated cosmetics, cosmetics or the like may be used as a base material. For example, materials such as animal or plant oil, mineral oil, ester oil, wax, higher alcohols, fatty acids, silicon oil, surfactant, phospholipids, alcohols, polyols, water-soluble polymers, clay minerals, purified water or the like, and if necessary, a pH regulator, an antioxidant, a chelating agent, an antiseptic, a fungicide, a colorant, an aromatic substance or the like may also be added. As to an inhaler, a compound having a ChE inhibitory activity, a salt thereof or a solvent thereof, or a prodrug thereof or a salt thereof or a solvent thereof can be delivered with an injector, a nebulizer, a pressurized package or other means suitable for delivering aerosol spray for inhalation administration. The pressurized package may contain an appropriate propellant. Moreover, for inhalation administration, a compound having a ChE inhibitory activity, a salt thereof or a solvate thereof, or a prodrug thereof, a salt thereof or a solvate thereof may be administered in a form of dry powdered composition or liquid spray. For administration with an adhesive patch via transdermal absorption, it is preferable to select a so-called free-form that does not form salt. For topical application to skin, a compound having a ChE inhibitory activity may be formulated into ointment, cream or lotion or as an active element for a transdermal patch. Ointment and cream can be formulated, for example, by adding an appropriate thickening agent and/or gelling agent to an aqueous or oil base. Lotion can be formulated by using an aqueous or oil base and may generally contain one or more of an emulsifying agent, a stabilizer, a dispersant, a suspending agent, a thickening agent and/or a colorant. The compound having a ChE inhibitory activity may also be administered by ion transfer therapy.

If necessary, components such as a blood circulating agent, a disinfectant, an anti-inflammatory agent, a cellular stimulant, vitamins, amino acids, a moisturizing agent, a keratolytic agent may further be blended. The proportion of the active principle to the carrier varies between 1 to 90% by weight.

The nerve regeneration stimulator of the present invention can generally include, as an active element, a compound having a ChE inhibitory activity, a salt thereof or a solvate thereof, or a prodrug thereof, a salt thereof or a solvate thereof at a proportion of 0.5% by weight or more, preferably 10 to 70% by weight.

When the compound having a ChE inhibitory activity, a salt thereof or a solvate thereof, or a prodrug thereof, a salt thereof or a solvate thereof is used for the treatment described above, it is purified for at least 90% or more, preferably 95% or more, more preferably 98% or more, still more preferably 99% or more.

A dose of the compound having a ChE inhibitory activity, a salt thereof or a solvate thereof, or a prodrug thereof, a salt thereof or a solvate thereof for oral administration varies as it is determined according to multiple factors including, for example, administration route, type of disease, degree of symptom, patient's age, sex and weight, type of salt, specific type of disease, pharmacological aspects such as pharmacokinetics and toxicological features, use of drug delivery system, and whether it is administered concomitantly with other drugs, but one skilled in the art will be able to determine appropriately. For example, for an adult (60 kg), about 0.001 to 1000 mg/day, preferably about 0.01 to 500 mg/day, and more preferably about 0.1 to 300 mg/day can be administered at one time or in several times. When administered to a child, a dose is possibly lower than that for an adult. The administration procedure actually used may widely vary and may depart from the preferable administration procedures described herein. For example, in the case of donepezil hydrochloride, preferably about 0.1 to 300 mg/day, more preferably about 0.1 to 100 mg/day, and still more preferably about 1.0 to 50 mg/day can be administered to an adult (weight 60 kg). In a preferred embodiment of donepezil hydrochloride, a 5 mg or 10 mg donepezil hydrochloride tablet commercially available under the trade name of Aricept tablet (Eisai Co., Ltd.), or donepezil hydrochloride under the trade name of Aricept fine granule (Eisai Co., Ltd.) can be administered. For example, tablets may be administered 1 to about 4 times a day. In a preferred embodiment, a 5 mg or 10 mg Aricept tablet (Eisai Co., Ltd.) is administered once a day. Those skilled in the art will appreciate that when donepezil hydrochloride is administered to a child, the dose thereof is possibly lower than that for an adult. In a preferred embodiment, donepezil hydrochloride can be administered to a child for about 0.5 to 10 mg/day, preferably about 1.0 to 3 mg/day. Preferably, in the case of Tacrine, about 0.1 to 300 mg/day, preferably about 40 to 120 mg/day is administered to an adult (weight 60 kg); in the case of Rivastigmine, about 0.1 to 300 mg/day, preferably about 3 to 12 mg/day is administered to an adult (weight 60 kg); in the case of galantamine, about 0.1 to 300 mg/day, preferably about 16 to 32 mg/day is administered to an adult (weight 60 kg); and in the case of physostigmine, about 0.1 to 300 mg/day, preferably about 0.6 to 24 mg/day is administered to an adult (weight 60 kg). For each of the above cases, a dose to a child may possibly be lower than that for an adult.

As to parenteral administration, a preferable dose for adhesive patch would be about 5 to 50 mg/day, more preferably about 10 to 20 mg/day for an adult (60 kg). An injectable agent may be produced by dissolving or suspending it in a pharmacologically acceptable carrier such as saline or a commercially available injectable distilled water to a concentration of 0.1 µg/ml carrier to 10 mg/ml carrier. A dose of the resulting injectable agent to a patient in need of the treatment may be about 0.01 to 50 mg/day, preferably about 0.01 to 5.0 mg/day, more preferably about 0.1 to 1.0 mg/day for an adult (60 kg), and may be administered 1 to 3 times a day. When administered to a child, the dose may possibly be lower than that for an adult.

### 3. Method for screening substance for stimulating nerve regeneration, pharmacologically acceptable salt thereof or solvate thereof

The present invention further provides a method for screening a substance that stimulates nerve regeneration, a pharmacologically acceptable salt thereof or a solvate thereof.

A screening process according to the present invention comprises administrating a candidate substance to neural stem cells, a tissue comprising neural stem cells or a non-human mammal comprising neural stem cells to act on said cells, and detecting or determining a change in a phenotype for nerve regeneration in the presence and absence of the candidate substance.

According to the screening process of the present invention, the candidate substance comprises a substance having a ChE (including AChE) inhibitory activity, for example, the compound having a ChE inhibitory activity described above, an anti-ChE antibody, siRNA and shRNA to ChE and the like. The substance may also be a salt thereof or a solvate of the above. The compound having a ChE inhibitory activity can be produced or obtained by referring to the description above. The anti-ChE antibody may be either a monoclonal antibody or a polyclonal antibody, and those skilled in the art would be able to produce such antibodies, for example, by using ChE as a sensitized antigen. siRNA or shRNA for ChE gene may be any nucleic acid that is capable of suppressing the expression of ChE gene, and those skilled in the art would be able to appropriately design and produce a sequence of siRNA or shRNA (Elbashir, S.M., et. al., Genes Dev., 15, 188-200, 2001).

A method for preparing neural stem cells may be carried out by a microsphere method (Cindi M Morshead and Derek van der Kooy, Cur. Opin. Neurobiol., 14, 125-131, 2004). A method for preparing a tissue comprising neural stem cells may be carried out by transcardiacly perfusing an animal under deep anesthesia with 4% paraformaldehyde in phosphate buffered saline (PBS) and slicing the removed brain into a thickness of 40 µm with a sliding microtome (Yoshimura et al., PNAS, 98, 5874-5879, 2001, Yoshimura et al., J. Clin. Invest., 112, 1202-1210, 2003). Examples of non-human mammals comprising neural stem cells include cow, monkey, avian, cat, mouse, rat, guinea pig, hamster, pig, dog and rabbit. Candidate compounds may be administered to said cells either orally or parenterally.

As an index of a phenotype change for nerve regeneration, at least one of the followings may be employed: proliferation ability of cells derived from the neural stem cells, differentiation potential of the cells, shape of the cells, motility of the cells, migration ability and ability to regulate migration of the cells, maturity of the cells, expression level of the functional protein, shape of the dendrites, shape of the axon, shape of the synapse, ability to form synapse, survival of the cells, retention of the cells and control of cell death. The substance may be determined to have an action of stimulating nerve regeneration when:
(a) the proliferation ability of cells derived from the neural stem cells, the differentiation potential of the cells, the motility of the cells, the migration ability and ability to regulate migration of the cells, the maturity of the cells, the expression level of the functional protein or the ability to form synapse is increased in the presence than in the absence of the test substance;
(b) the shape of the cells, the shape of the dendrites, the shape of the axon or the shape of the synapse is more amenable to functional expression in the presence than in the absence of the test substance; or
(c) the degree of survival of the cells, retention of the cells or control of cell death is increased in the presence than in the absence of the test substance.

In order to detect or determine the change in a phenotype of cells derived from neural stem cells, a labeling substance (e.g., BrdU, retrovirus vector, etc.) that is incorporated upon the cell division of neural stem cells may be used as a label marker. The phenotype change for the nerve regeneration may be detected or determined by observing the labeling substance by immunostaining technique. For example, the amount of BrdU uptake upon action of the test substance on the cells in the presence of BrdU may be an index in the method for screening a substance that stimulates nerve regeneration. BrdU may be incorporated into the cells through contact therewith at a concentration of 0.1 to 1000 mg/kg per time, every 0.5 to 24 hours for 1 to 10 times. The amount of BrdU uptake may be detected by using an antibody to BrdU (see Examples 2 and 3). An example of a method for detecting or determining a change in the phenotype for the nerve regeneration includes double staining using the above-mentioned labeling substance for neural stem cells together with the index of the nerve regeneration phenotype.

The present invention further provides a kit for screening a substance having an action of stimulating nerve regeneration, a pharmacologically acceptable salt thereof or a solvate thereof that are to be used in the method described above. The screening kit of the invention comprises means required for determining a change in the phenotype for nerve regeneration. Suitably, reagents used upon determining the change in nerve regeneration phenotype are those used in Examples 2 and 3 below. The screening kit of the present invention may further include an instruction, a tube, a flask or the like.

### EXAMPLES

Hereinafter, the present invention will be described more specifically by way of non-limiting examples.

### EXAMPLE 1

### Production of donepezil hydrochloride

### (a) Synthesis of 1-benzyl-4-piperidine carboaldehyde

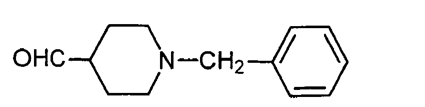

26.0 g of methoxymethylenetriphenylphosphonium chloride was suspended in 200 ml anhydrous ether, and 1.6 M n-butyllithiumhexane solution was added dropwise at room temperature. After stirring at room temperature for 30 minutes, the resultant was cooled to 0°C, and 14.35 g 1-benzyl-4-piperidone in 30 ml anhydrous ether solution was added. After stirring at room temperature for 3 hours, insoluble matter was filtered out and the filtrate was concentrated under reduced pressure. The obtained residue was dissolved in ether and extracted with IN hydrochloric acid. After adjusting pH to 12 with aqueous sodium hydroxide solution, the resultant was extracted with methylene chloride. The resultant was dried with magnesium sulfate, and concentrated under reduced pressure. The obtained residue was purified through a silica gel column, thereby obtaining 5.50 g of oily substance (yield 33%).

Subsequently, the obtained oily substance was dissolved in 40 ml methanol, and added with 40 ml 1N hydrochloric acid. The reaction solution was heated to reflux for 3 hours, then concentrated under reduced pressure. The residue was dissolved in water. Thereafter, pH of the dissolved solution was adjusted to 12 with aqueous sodium hydroxide solution, and extracted with methylene chloride. The extracted solution was washed with saturated saline, dried with magnesium sulfate, and concentrated under reduced pressure. The obtained residue was purified through a silica gel column to obtain 2.77 g of 1-benzyl-4-piperidinecarboaldehyde (yield 54%) as an oily substance.

The structure of the obtained compound was determined by NMR.

Molecular formula; C₁₃H₁₇NO ¹H-NMR (CDCl₃)δ; 1.40-2.40 (7H, m), 2.78 (2H, dt), 3.45 (2H, s), 7.20 (5H, s), 9.51 (1H, d).

### (b) Synthesis of 1-benzyl-4-[(5,6-dimethoxy-1-indanone)-2-ylidenyl]methylpiperidine hydrochloride

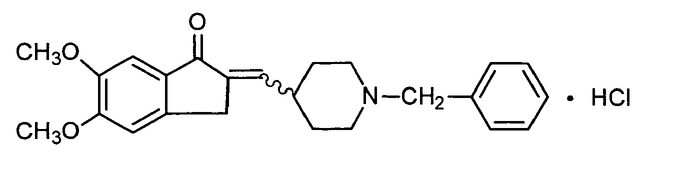

This reaction took place in argon atmosphere.

2.05 ml diisopropylamine was added to 10 ml anhydrous THF and 9.12 ml 1.6M n-butyllithium hexane solution was further added at 0°C. The resultant was stirred at 0°C for 10 minutes, cooled to -78°C, and added with 2.55 g 5,6-dimethoxy-1-indanone in 30 ml anhydrous THF solution and 2.31 ml hexamethyl phosphoramide. The resultant was stirred at -78°C for 15 minutes, added with 2.70 g 1-benzyl-4-piperidinecarboaldehyde obtained in (a) in 30 ml anhydrous THF solution, and gradually heated to room temperature. Again stirring at room temperature for another 2 hours, 1% aqueous ammonium chloride solution was added to separate the organic layer. Next, the aqueous layer was extracted with ethyl acetate, combined with the organic layer separated above, and washed with saturated saline. The solution was dried with magnesium sulfate, concentrated under reduced pressure, and the obtained residue was purified through a silica gel column (methylene chloride: methanol = 500:1 to 100:1). After concentrating the eluate under reduced pressure, the resultant was dissolved in methylene chloride, added with 10% hydrochloric acid-ethyl acetate solution, and further concentrated under reduced pressure to obtain a crystal. This was recrystallized from methylene chloride-IPE to obtain 3.40 g of 1-benzyl-4-[(5,6-dimethoxy-1-indanone)-2-ylidenyl]methylpiperidine hydrochloride (yield 62%) having the following properties:
- Melting point (°C); 237-238 (dec.)
- Elementary analysis; as C₂₄H₂₇NO₃ HCl, CHN calculated (%): 69.64 6.82 3.38, found (%): 69.51 6.78 3.30.

### (c) 1-benzyl-4-[(5,6-dimethoxy-1-indanone)-2-yl]methylpiperidine hydrochloride

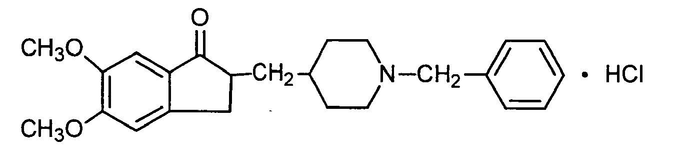

0.40 g of 1-benzyl-4-[(5,6-dimethoxy-1-indanone)-2-ylidenyl]methylpiperidine obtained in (b) was dissolved in 16 ml THF, and 0.04 g of 10% palladium-carbon was added. After hydrogenating under atmospheric pressure at room temperature for 6 hours, catalyst was filtered out, and the filtrate was concentrated under reduced pressure. The residue was purified through a silica gel column (methylene chloride: methanol=50:1), and the eluate was concentrated under reduced pressure. Thereafter, the residue was dissolved in methylene chloride, added with 10% hydrochloric acid-ethyl acetate solution, and was further concentrated under reduced pressure, thereby obtaining a crystal. This was recrystallized from ethanol-IPE to obtain 0.36 g of 1-benzyl-4-[(5,6-dimethoxy-1-indanone)-2-yl]methylpiperidine hydrochloride (donepezil hydrochloride) (yield 82%) having the following properties:
- Melting point (°C); 211-212 (dec.)
- Elementary analysis; as C₂₄H₂₉NO₃ HCl, CHN calculated (%): 69.30 7.27 3.37, found (%): 69.33 7.15 3.22.

### EXAMPLE 2 Stimulating effect of donepezil on nerve regeneration in mouse hippocampal dentate gyrus

### (1) Animal breeding

Animal breeding was carried out according to "Guide for the Care and Use of Laboratory Animals" SOP No. GHZ-3001. C57BL/6 male SPF mice (8-week-old at the beginning of the experiments) purchased from Japan SLC, Inc. were used. Animals were subjected to experiments after acclimating in a breeding room under control at a temperature of 23 ± 1°C and illumination of 12 hours (7:00 to 19:00) for 5 days or longer. During breeding, animals had free access to solid food MF (Oriental Yeast Co., Ltd.) and water (tap water).

### (2) Administration of BrdU and donepezil

BrdU (Sigma) for labeling neural stem cells was used by dissolving in a phosphate buffer solution (PBS: Sigma) containing 0.007N HCl at a concentration of 5 mg/mL (Yoshimura et al., PNAS, 98, 5874-5879, 2001, Yoshimura et al., J. Clin. Invest., 112, 1202-1210, 2003). BrdU was intraperitoneally administered at a dose of 50 mg/kg (0.1 ml BrdU solution/10g weight) for three times (every 4 hours).

Two groups, i.e., vehicle control group and donepezil administered group, were prepared. Donepezil hydrochloride obtained in Example 1 was used. Donepezil was dissolved in injectable distilled water (Otsuka Pharmaceutical Co., Ltd.) (vehicle) upon use. Donepezil was orally administered at a dose of 1 mg/kg (0.1 ml donepezil solution/10g weight) four hours after the final BrdU administration. Subsequently, donepezil was orally administered at a dose of 1 mg/kg at night once a day for 14 days. Injectable distilled water was orally administered to animals of the vehicle control group.

### (3) Immunohistochemical detection of BrdU

For immunohistochemical assessment, on the day following the final donepezil administration, the animals were transcardiacly perfused with 4% paraformaldehyde in phosphate buffered saline (PBS) solution under deep anesthesia of urethane (Sigma). The removed brain was sliced into coronal sections in a frozen state with a thickness of 40 µm using a sliding microtome (Leica, Model SM2000R, Nussloch). The sliced brain tissues were successively placed in 10 wells of a culture plate with a PBS solution containing sodium azide such that 10 sections were placed per well.

Immunostaining of the brain tissues were carried out by flotation (Yoshimura et al., PNAS, 98, 5874-5879, 2001, Yoshimura et al., J. Clin. Invest., 112, 1202-1210, 2003). Peroxidase method (ABC system, Vector Laboratories Inc.) was used for immunohistochemical detection of BrdU (Yoshimura et al., PNAS, 98, 5874-5879, 2001, Yoshimura et al., J. Clin. Invest., 112, 1202-1210, 2003). As a primary antibody to BrdU, rat anti-BrdU antibody (400-fold-diluted, Accurate Chemical & Scientific Corporation) was used. As a secondary antibody, rabbit IgG biotinylated antibody (200-fold-diluted, Vector Laboratories Inc.) was used

### (4) Quantitation of number of BrdU positive cells

For quantitation, four hippocampal sections (intervals of sections being 400 µm, four sections between -1.4 mm to -2.6 mm from bregma) were used for each individual. All of the BrdU positive cells present in a subgranular zone (SGZ) and a granule cell layer (GCL) of the hippocampal dentate gyrus were determined. SGZ was defined as an area with a depth of 20 µm (a size corresponding to two cells) just below the GCL (on the side of hylus). GCL was defined as a granule nerve cell layer that was positive to cresyl violet staining. The GCL area in each hippocampal section was determined as a cresyl violet positive GCL area using an image analyzing software (NIH, image ver. 1.60).

A volume of GCL (GCLV) was determined as a GCL volume in left hemisphere between -1.4 mm to -2.6 mm from bregma (Cavalieri technique). The number of BrdU positive cells in GCLV was calculated by multiplying the number of BrdU positive cells in the four hippocampal sections by 10. This value was assumed as the number of BrdU positive cells in each hippocampal dentate gyrus (Yoshimura et al., J. Clin. Invest., 112, 1202-1210, 2003).

### (5) Statistical analysis

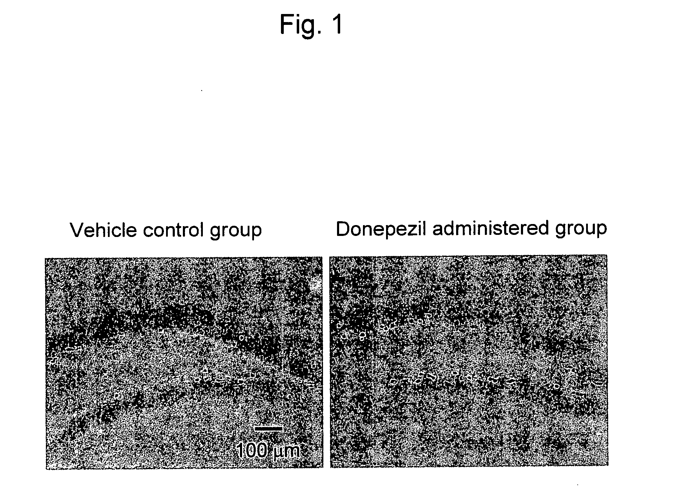

All of the quantitative values are indicated in mean ± standard error (n = 8 for each group). Statistical significance between the vehicle control group and the donepezil group was analyzed by unpaired t test where significance level was 5% (two-sided). The results are shown in Table 1 and Figure 1. In Table 1, "*" indicates p < 0.05, in comparison to the vehicle control group.

**Table 1**

| Number of BrdU positive cells (mice) after donepezil treatment | | |
|---|---|---|
| | Vehicle control group | Donepezil administered group |
| GCL volume (mm³) | 0.189 ± 0.006 | 0.195 ± 0.004 |
| Number of BrdU positive cells per hippocampus | 1091 ± 56 | 1528 ± 137* |

There was no difference of the GCL volume between the vehicle control group and the donepezil administered group (Table 1). This shows that donepezil has no effect on the volume of hippocampal nerve cell layer under these administration conditions. On the other hand, the number of BrdU positive cells per hippocampus was higher for the donepezil administered group than the vehicle group, which was statistically significant (Table 1, Figure 1). Thus, donepezil was proved to retain cells regenerating from neural stem cells (BrdU positive cells) present in dentate gyrus.

### EXAMPLE 3: Stimulating effect of donepezil on nerve regeneration in rat hippocampal dentate gyrus

### (1) Animal breeding

SD male SPF rats (6-week-old at the beginning of the experiments) purchased from Japan SLC, Inc. were bred under the same conditions as the mice in Example 2. Animals were subjected to experiments after acclimating for 5 days or longer following purchase.

### (2) Administration of BrdU and donepezil

BrdU (Sigma) for labeling neural stem cells was dissolved in PBS containing 0.014N HCl at a concentration of 10 mg/mL. BrdU was intraperitoneally administered at a dose of 50 mg/kg for three times (every 4 hours).

Three groups, i.e., vehicle control group and donepezil administered groups, (0.5 and 2 mg/kg) were prepared. Donepezil specimen used in Example 2 was used. Donepezil was dissolved in injectable distilled water to 0.4 mg/ml (2 mg/kg group) or 0.1 mg/ml (0.5 mg/kg group) and kept frozen until administration. Donepezil was orally administered at a dose of 0.5 and 2 mg/kg four hours after the final BrdU administration at 5 ml/kg weight. Subsequently, donepezil was orally administered at the same dose once a day at night for 28 days. Injectable distilled water was orally administered at the same volume to animals in the vehicle control group.

### (3) Immunohistochemical detection of BrdU

For immunohistochemical assessment, on the day following the final donepezil administration, the animals were transcardiacly perfused with 4% paraformaldehyde in PBS solution under deep anesthesia of Nembutal. The removed brain was sliced into sections as described in Example 2 for use in immunohistochemical studies.

Immunostaining of the brain tissues were carried out as described in Example 2.

### (4) Quantitation of number of BrdU positive cells

For quantitation, four hippocampal sections (thickness of 40µm, intervals of sections being 400 µm, four sections between -3.6 mm to -4.8 mm from bregma) were used for each individual. BrdU positive cells were counted in the same manner as Example 2. The GCL volume in each hippocampal section and the total number of BrdU positive cells in each hippocampal dentate gyrus were also determined as described in Example 2.

### (5) Statistical analysis

All of the quantitative values are indicated in mean ± standard error (vehicle control group n = 12, donepezil administered group n = 11). Statistical significance between the vehicle control group and each donepezil group was analyzed by one-way analysis of variance followed by Dunnett multiple comparison where significance level was 5% (two-sided). The results are shown in Table 2. In Table 2, "*" indicates p < 0.05, in comparison to the vehicle control group.

**Table 2**

| Number of BrdU positive cells (rats) after donepezil treatment | | | |
|---|---|---|---|
| | Vehicle control group | Donepezil administered group | |
| | | 0.5 mg/kg | 2 mg/kg |
| GCL volume (mm³) | 1.30 ±0.05 | 1.27 ±0.04 | 1.35 ±0.04 |
| Number of BrdU positive cells per hippocampus | 5490 ±179 | 6880 ± 436* | 6694 ±357* |

There was no difference of the GCL volume between the vehicle control group and each donepezil administered group (Table 2). This shows that donepezil has no influence on the volume of rat hippocampal nerve cell layer under these administration conditions. On the other hand, the number of BrdU positive cells per hippocampus was higher for each donepezil administered group than the vehicle group, which was statistically significant (Table 2). Thus, donepezil was proved to retain cells (BrdU positive cells) regenerating from neural stem cells present in rat dentate gyrus.

### EXAMPLE 4: Expression of acetylcholine receptor in newborn nerve in rat hippocampal dentate gyrus

The brain sections of vehicle control group obtained in Example 3 were used to examine the expression of acetylcholine receptor (muscarine M2 receptor) in BrdU positive cells of the hippocampal dentate gyrus. Procedure from the beginning to the preparation of the brain sections was the same as Examples 2 and 3. Staining of BrdU was carried out using mouse anti-BrdU IgG (Becton Dickinson, 200 fold) as the primary antibody and RRX-labeled goat anti-mouse IgG (Vector, 200 fold) as the secondary antibody. The muscarine M2 receptor was detected by using rabbit anti-muscarine M2 receptor IgG (ALOMONE Labs, 300 fold) as the primary antibody and biotin-labeled goat anti-rabbit IgG (Vector, 200 fold) + cy2-labeled streptavidin (Jackson, 200 fold) as the secondary antibody.

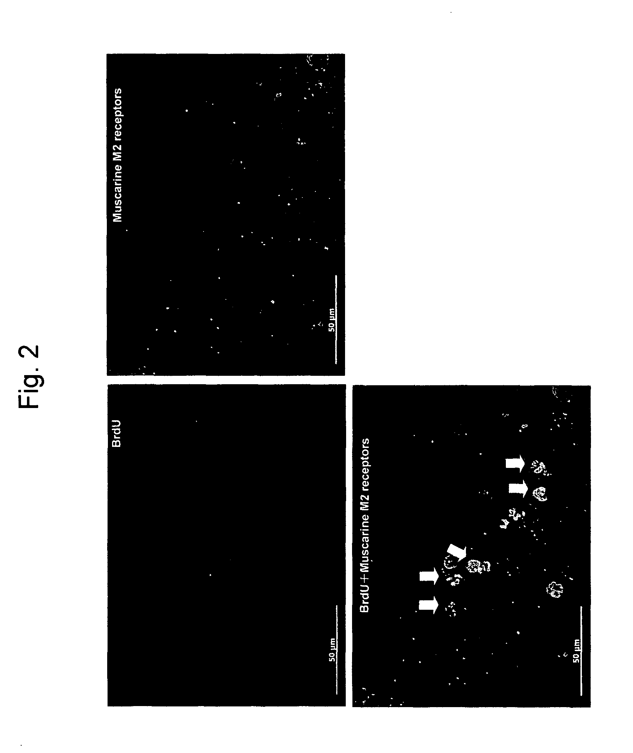

As shown in Figure 2, muscarine M2 receptor (green fluorescence) was found to be expressed on the BrdU positive cells (red fluorescence) present in GCL of hippocampal dentate gyrus.

### EXAMPLE 5: Increase in intracellular calcium concentration of fetal rat neural progenitor cells by acetylcholine receptor stimulation

### (1) Preparation of neural progenitor cells

Cerebrums were isolated from fetuses obtained from 14.5 days pregnant SD female rats (Japan SLC, Inc.). Cells were dispersed by pipetting the tissue in cold L-15 medium. These cells were cultured in a suspension containing 20 rig/ml human basic fibroblast growth factor (bFGF) in serum-free D-MEM/F-12 medium (supplemented with B-27, N2 (both from Invitrogen) and gentamicin beforehand). The rat neural progenitor cells are known to form a neurosphere in this medium. After 5 to 7 days, the neurosphere was collected and pipetted to isolate the rat neural progenitor cells, followed by passage culture using the medium described above.

### (2) Measurement of intracellular calcium (Ca⁺⁺) concentration

The neural progenitor cells were isolated from the neurosphere that was collected a day before the experiment and suspended in the bFGF-free medium otherwise same as described above. After the number of cells was counted, 50,000 cells each were suspended in 100 µL of the medium described above and dispensed into a 96-flat bottom culture plate. Intracellular Ca⁺⁺ concentration was determined using a commercially available kit (Calcium Kit-Fluo-3, Dojindo laboratories) according to the attached instruction. To some of the wells, atropine (muscarine receptor antagonist) was added to 25 µmol/L and cultured at 37°C for 30 minutes beforehand. Thereafter, acetylcholine agonist (acetylcholine or carbachol) was added to 25 µmol/L. The change in the intracellular Ca⁺⁺ concentration was determined as a change in the fluorescent intensity of Fluo-3 every second in each well using a fluorescence image analyzer (FDSS6000, Hamamatsu Photonics KK) (excitation filter 480 nm, fluorescence filter 520-560 nm).

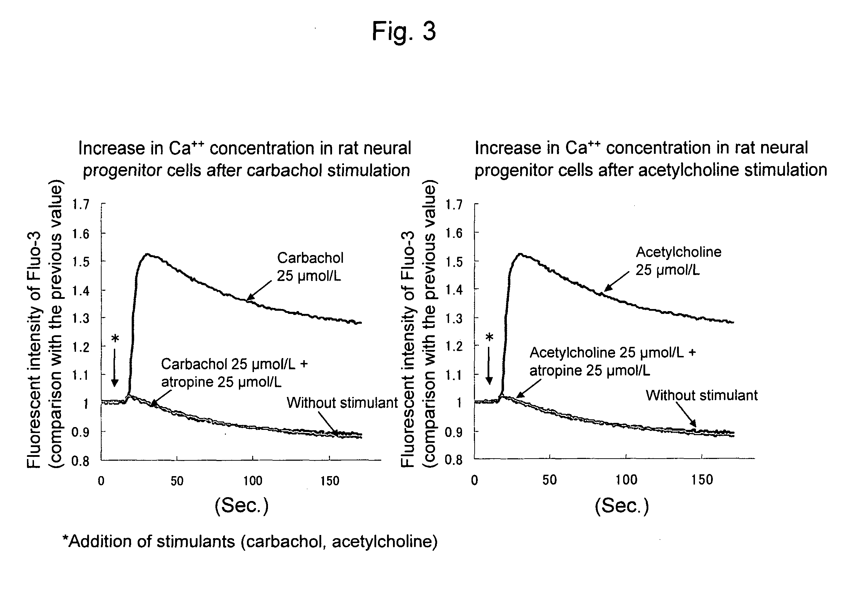

As shown in Figure 3, stimulation with acetylcholine (25 µmol/L) and carbachol (25 µmol/L) increased intracellular Ca⁺⁺ concentration of the rat neural progenitor cell specimens. Since the effects of all drugs were completely antagonized by atropine pretreatment, this reaction was shown to have occurred via the muscarine receptor.

### EXAMPLE 6: Detection of acetylcholine receptor on fetal rat neural progenitor cells

### (1) BrdU labeling of rat neural progenitor cells

Rat neural progenitor cells prepared in the same manner as Example 5 was suspended in the bFGF-free medium described above at 200,000 cells/mL. To the resultant, BrdU (Sigma) was added to a final concentration of 10 µmol/L and cultured at 37°C under 5% CO₂ for 4 hours. Through this process, the neural progenitor cells undergoing DNA replication present in the cell specimens are labeled. The cultured cells were collected, washed, suspended in the same medium and dispensed for 200,000 each in a 24-well culture plate covered with a glass cover (precoated with poly-L-ornithine, fibronectin). After an overnight culture, the cells were fixed with 4% paraformaldehyde.

### (2) Detection of acetylcholine receptor by immunostaining

The fixed cells described above was treated with 0.3%Triton X-100 and washed with PBS. Each specimen was treated with 2N hydrochloric acid at 37°C for 30 minutes, followed by washing twice with a boric acid buffer. The resultant was allowed to stand still in the presence of a primary antibody diluted with 10% goat serum in PBS (GS-PBS) (rabbit anti-muscarine M2 receptor IgG (ALOMONE Labs, 200 fold)) at 4°C overnight. After washing with 0.1% Triton X-100 in PBS (T-PBS), a secondary antibody diluted with GS-PBS (Alexa 488-labeled goat anti-rabbit IgG, Molecular Probe, 5000 fold) was added and allowed to stand still at room temperature for an hour. In addition, BrdU incorporated into the cells were stained using Alexa 546-labeled anti-BrdU antibody (Molecular Probe, 250 fold). The cell specimens after staining were observed using a fluorescence microscope (Carl Zeiss, Axiovert200M).

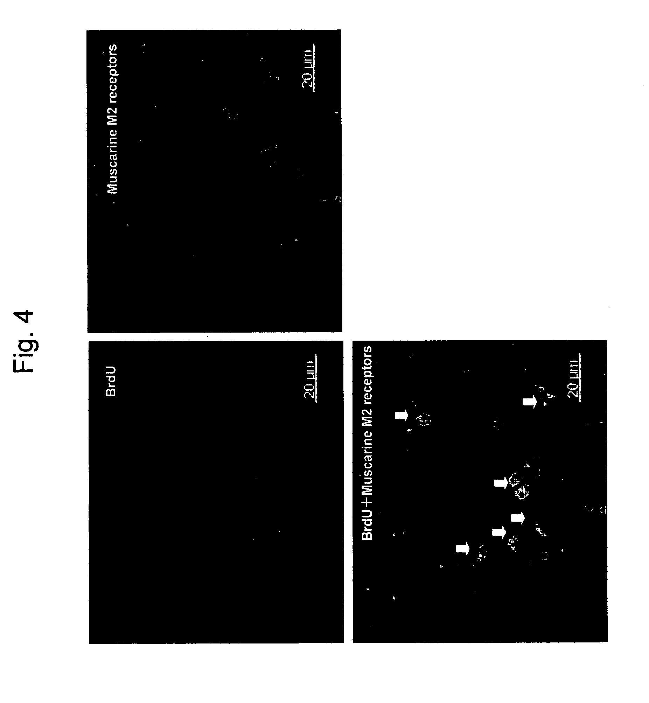

As a result of the observation, expression of muscarine M2 receptor (green fluorescence) was confirmed on the proliferating rat neural progenitor cells that were BrdU positive (red fluorescence) (Figure 4). Therefore, expression of functional acetylcholine receptors on the rat neural progenitor cells became apparent.

### INDUSTRIAL APPLICABILITY

The present invention provides a nerve regeneration stimulator comprising, as an active element, a compound having a cholinesterase (ChE) inhibitory activity, a pharmacologically acceptable salt thereof or a solvate thereof and provides a screening method thereof. The nerve regeneration stimulator of the invention and a compound obtained by the screening method of the invention are useful as a novel agent for treating diseases associated with damage of hippocampal nerve function.

## Claims

1. A nerve regeneration stimulator comprising a compound with a cholinesterase inhibitory activity, a pharmacologically acceptable salt thereof or a solvate thereof.

2. A nerve regeneration stimulator according to Claim 1, wherein the compound with a cholinesterase inhibitory activity is a cyclic amine derivative represented by the following general formula: 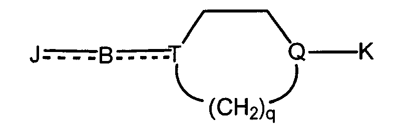 (wherein, J is:
(a) a substituted or unsubstituted (1) phenyl group, (2) pyridyl group, (3) pyradyl group, (4) quinolyl group, (5) cyclohexyl group, (6) quinoxalyl group or (7) furyl group;
(b) a monovalent or divalent group derived from a group selected from the group consisting of (1) indanyl, (2) indanonyl, (3) indenyl, (4) indenonyl, (5) indandionyl, (6) tetralonyl, (7) benzsuberonyl, (8) indanolyl and (9) a group represented by formula 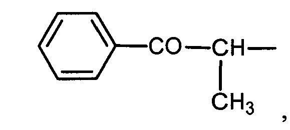 in all of which a phenyl group may be substituted;
(c) a monovalent group derived from a cyclic amide compound;
(d) a lower alkyl group; or
(e) a group represented by formula R¹-CH = CH- (wherein R¹ is a hydrogen atom or a lower alkoxycarbonyl group),
B is a group represented by formula 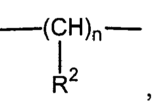 a group represented by formula 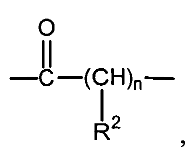 a group represented by formula 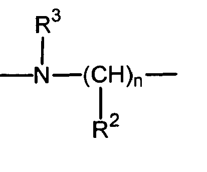 (wherein, R³ is a hydrogen atom, a lower alkyl group, an acyl group, a lower alkylsulfonyl group, a substituted or unsubstituted phenyl group or a benzyl group), a group represented by formula 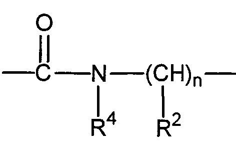 (wherein, R⁴ is a hydrogen atom, a lower alkyl group or a phenyl group), a group represented by formula 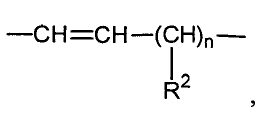 a group represented by formula 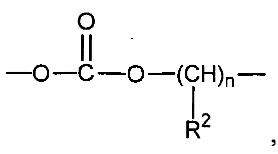 a group represented by formula 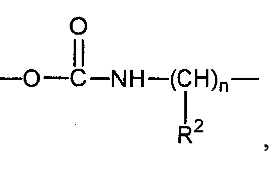 a group represented by formula 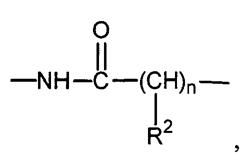 a group represented by formula 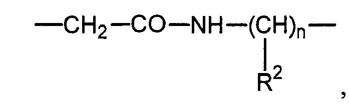 a group represented by formula 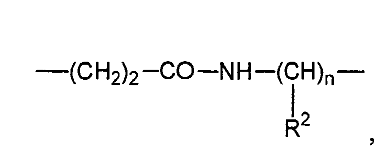 a group represented by formula 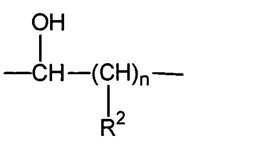 (wherein, n is 0 or an integer of 1 to 10, and R² is a hydrogen atom or a methyl group), a group represented by formula =(CH-CH=CH)_{b}- (wherein, b is an integer of 1 to 3), a group represented by formula =CH-(CH₂)_{c}- (wherein, c is 0 or an integer of 1 to 9), a group represented by formula =(CH-CH)_{d}= (wherein, d is 0 or an integer of 1 to 5), a group represented by formula 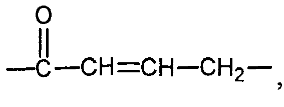 a group represented by formula 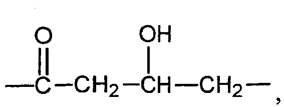 a group represented by formula 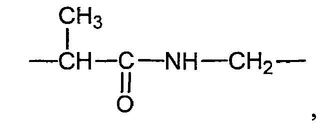 a group represented by formula 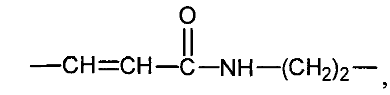 a group represented by formula -NH-, a group represented by formula -O-, a group represented by formula -S-, a dialkylaminoalkylcarbonyl group or a lower alkoxycarbonyl group,
T is a nitrogen atom or a carbon atom,
Q is a nitrogen atom, a carbon atom or a group represented by formula 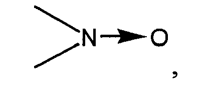 K is a hydrogen atom, a substituted or unsubstituted phenyl group, an arylalkyl group in which a phenyl group may be substituted, a cinnamyl group in which a phenyl group may be substituted, a lower alkyl group, a pyridylmethyl group, a cycloalkylalkyl group, an adamantanemethyl group, a furylmethyl group, a substituted or unsubstituted cycloalkyl group, a lower alkoxycarbonyl group or an acyl group,
q is an integer of 1 to 3, and
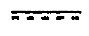 indicates a single bond or a double bond).

3. A nerve regeneration stimulator according to Claim 2, wherein J is a group selected from the group consisting of: substituted or unsubstituted (1) phenyl group, (2) pyridyl group, (3) pyradyl group, (4) quinolyl group, (5) cyclohexyl group, (6) quinoxalyl group and (7) furyl group.

4. A nerve regeneration stimulator according to Claim 2, wherein J is a monovalent group derived from a cyclic amide compound.

5. A nerve regeneration stimulator according to Claim 1, wherein the compound having a cholinesterase inhibitory activity is a cyclic amine derivative represented by the following general formula: 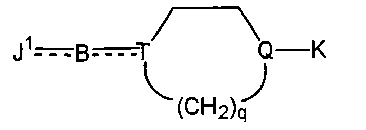 (wherein, J¹ is a monovalent or divalent group derived from a group selected from the group consisting of (1) indanyl, (2) indanonyl, (3) indenyl, (4) indenonyl, (5) indandionyl, (6) tetralonyl, (7) benzsuberonyl, (8) indanolyl and (9) a group represented by formula 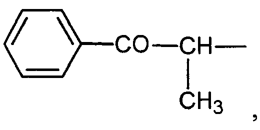 in all of which a phenyl group may be substituted,
B is a group represented by formula 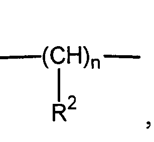 a group represented by formula 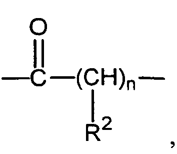 a group represented by formula 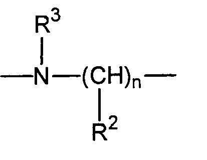 (wherein, R³ is a hydrogen atom, a lower alkyl group, an acyl group, a lower alkylsulfonyl group, a substituted or unsubstituted phenyl group or a benzyl group), a group represented by formula 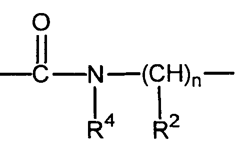 (wherein, R⁴ is a hydrogen atom, a lower alkyl group or a phenyl group), a group represented by formula 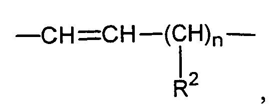 a group represented by formula 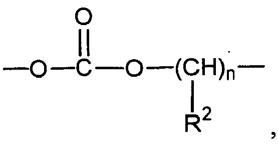 a group represented by formula 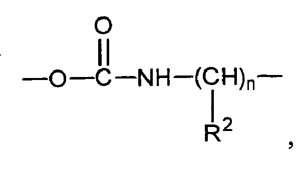 a group represented by formula 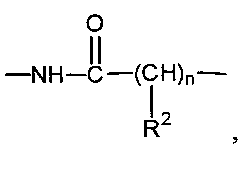 a group represented by formula 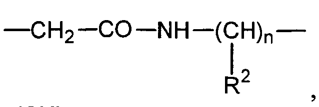 a group represented by formula 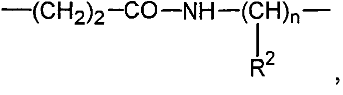 a group represented by formula 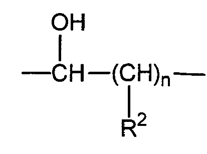 (wherein, n is 0 or an integer of 1 to 10, and R² is a hydrogen atom or a methyl group), a group represented by formula =(CH-CH=CH)_{b}- (wherein, b is an integer of 1 to 3), a group represented by formula =CH-(CH₂)_{c}- (wherein, c is 0 or an integer of 1 to 9), a group represented by formula =(CH-CH)_{d}= (wherein, d is 0 or an integer of 1 to 5), a group represented by formula 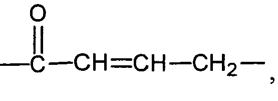 a group represented by formula 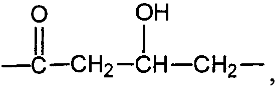 a group represented by formula 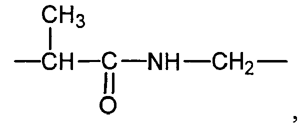 a group represented by formula 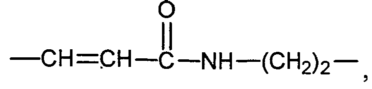 a group represented by formula -NH-, a group represented by formula -O-, a group represented by formula -S-, a dialkylaminoalkylcarbonyl group or a lower alkoxycarbonyl group,
T is a nitrogen atom or a carbon atom,
Q is a nitrogen atom, a carbon atom or a group represented by formula 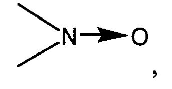 K is a hydrogen atom, a substituted or unsubstituted phenyl group, an arylalkyl group in which a phenyl group may be substituted, a cinnamyl group in which a phenyl group may be substituted, a lower alkyl group, a pyridylmethyl group, a cycloalkylalkyl group, an adamantanemethyl group, a furylmethyl group, a substituted or unsubstituted cycloalkyl group, a lower alkoxycarbonyl group or an acyl group,
q is an integer of 1 to 3, and
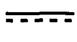 indicates a single bond or a double bond).

6. A nerve regeneration stimulator according to Claim 5, wherein B is a group represented by formula 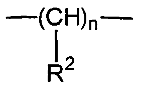 (wherein n is 0 or an integer of 1 to 10, and R² is a hydrogen atom or a methyl group), a group represented by formula -CH=CH-(CH)ₙR²- (wherein, n is 0 or an integer of 1 to 10, and R² is a hydrogen atom or a methyl group), a group represented by formula =(CH-CH=CH)_{b}- (wherein, b is an integer of 1 to 3), a group represented by formula =CH-(CH₂)_{c}- (wherein, c is 0 or an integer of 1 to 9) or a group represented by formula =(CH-CH)_{d}= (wherein, d is 0 or an integer of 1 to 5).

7. A nerve regeneration stimulator according to Claim 1, wherein the compound having a cholinesterase inhibitory activity is a cyclic amine derivative represented by the following general formula: 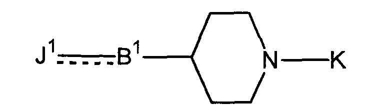 (wherein, J¹ is a monovalent or divalent group derived from a group selected from the group consisting of (1) indanyl, (2) indanonyl, (3) indenyl, (4) indenonyl, (5) indandionyl, (6) tetralonyl, (7) benzsuberonyl, (8) indanolyl and (9) a group represented by formula 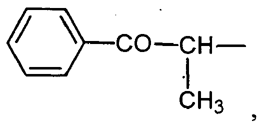 in all of which a phenyl group may be substituted,
B¹ is a group represented by formula 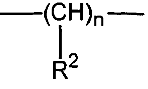 (wherein, n is 0 or an integer of 1 to 10, and R² is a hydrogen atom or a methyl group), a group represented by formula -CH=CH-(CH)ₙR²- (wherein, n is 0 or an integer of 1 to 10, and R² is a hydrogen atom or a methyl group), a group represented by formula =(CH-CH=CH)_{b}-(wherein, b is an integer of 1 to 3), a group represented by formula =CH-(CH₂)_{c}-(wherein, c is 0 or an integer of 1 to 9) or a group represented by formula =(CH-CH)_{d}= (wherein, d is 0 or an integer of 1 to 5), and
K is a hydrogen atom, a substituted or unsubstituted phenyl group, an arylalkyl group in which a phenyl group may be substituted, a cinnamyl group in which a phenyl group may be substituted, a lower alkyl group, a pyridylmethyl group, a cycloalkylalkyl group, an adamantanemethyl group, a furylmethyl group, a substituted or unsubstituted cycloalkyl group, a lower alkoxycarbonyl group or an acyl group).

8. A nerve regeneration stimulator according to Claim 7, wherein K is a substituted or unsubstituted arylalkyl group or phenyl group.

9. A nerve regeneration stimulator according to either one of Claims 7 and 8, wherein J¹ is a group selected from the group consisting of monovalent and divalent groups derived from indanonyl, indenyl and indanedionyl.

10. A nerve regeneration stimulator according to either one of Claims 7 and 8, wherein J¹ is an indanonyl group that may contain, as a substituent, a lower alkyl group with a carbon number 1 to 6 or a lower alkoxy group with a carbon number 1 to 6.

11. A nerve regeneration stimulator according to Claim 2, wherein the cyclic amine derivative is at least one selected from the group consisting of:
1-benzyl-4-((5,6-dimethoxy-1-indanone)-2-yl)methylpiperidine,
1-benzyl-4-((5,6-dimethoxy-1-indanone)-2-ylidenyl)methylpiperidine,
1-benzyl-4-((5 -methoxy-1-indanone)-2-yl)methylpiperidine,
1-benzyl-4-((5,6-methylenedioxy-1-indanone)-2-yl)methylpiperidine, 1-(m-nitrobenzyl)-4-((5,6-dimethoxy-1-indanone)-2-yl)methylpiperidine,
1-cyclohexylmethyl-4-((5,6-dimethoxy-1-indanone)-2-yl)methylpiperidine,
1-(m-fluorobenzyl)-4-((5,6-dimethoxy-1-indanone)-2-yl)methylpiperidine,
1-benzyl-4-(3-((5,6-dimethoxy-1-indanone)-2-yl)propyl)piperidine,
1-benzyl-4-((5-isopropoxy-6-methoxy-1-indanone)-2-yl)methylpiperidine,
1-benzyl-4-((5,6-dimethoxy-1-indanone)-2-ylidenyl)propenylpiperidine, and
1-benzyl-4-((5,6-dimethoxy-1,3-indandione)-2-yl)propenylpiperidine.

12. A nerve regeneration stimulator according to Claim 2, wherein the cyclic amine derivative is 1-benzyl-4-((5,6-dimethoxy-1-indanone)-2-yl)methylpiperidine.

13. A nerve regeneration stimulator according to Claim 1, wherein the compound having a cholinesterase inhibitory activity is
1-benzyl-4-((5,6-dimethoxy-1-indanone)-2-yl) methylpiperidine hydrochloride.

14. A nerve regeneration stimulator according to Claim 1, wherein the compound having a cholinesterase inhibitory activity is galantamine, tacrine, physostigmine or rivastigmine.

15. A method for screening a substance that stimulates nerve regeneration, comprising: administering a test substance to neural stem cells, a tissue comprising neural stem cells or a non-human mammal; and detecting or determining a change in the phenotype for nerve regeneration in the presence and in the absence of the candidate substance.

16. A method according to Claim 15, wherein the candidate substance is a compound having a cholinesterase inhibitory activity, a pharmacologically acceptable salt thereof or a solvate thereof.

17. A method according to Claim 16, wherein the compound having a cholinesterase inhibitory activity is a compound having an acetylcholinesterase inhibitory activity, a pharmacologically acceptable salt thereof or a solvate thereof.

18. A screening method according to any one of Claims 15 to 17, wherein the phenotype change for nerve regeneration comprises, as an index, at least one selected from the group consisting of proliferation ability of cells derived from the neural stem cells, differentiation potential of the cells, shape of the cells, motility of the cells, migration ability and ability to regulate migration of the cells, maturity of the cells, expression level of the functional protein, shape of the dendrites, shape of the axon, shape of the synapse, ability to form synapse, survival of the cells, retention of the cells and control of cell death.

19. A method for stimulating nerve regeneration, comprising administering to a patient a compound having a cholinesterase inhibitory activity according to any one of Claims 1 to 14, a pharmacologically acceptable salt thereof or a solvate thereof.
